# EUROPÄISCHE PATENTSCHRIFT

(11) **EP 1 363 543 B1**
(45) Veröffentlichungstag und Bekanntmachung des Hinweises auf die Patenterteilung: **06.09.2006**
(21) Anmeldenummer: 02700094.2
(22) Anmeldetag: 04.03.2002
(51) Int. Cl.: A61B 17/68, A61B 17/88

(54) **Implantate und Vorrichtung zum Verbinden von Gewebeteilen**
Implants and device for joining tissue parts
Implants et dispositif permettant de relier des parties de tissu

(30) Priorität: 02.03.2001 CH 387012001
(43) Veröffentlichungstag der Anmeldung: 26.11.2003
(73) Patentinhaber: Woodwelding AG, 8002 Zürich (CH)
(72) Erfinder: AESCHLIMANN, Marcel, CH-2514 Ligerz (CH); TORRIANI, Laurent, CH-2502 Biel (CH); LANCI, Antonino, CH-3006 Bern (CH); MAYER, Jörg, CH-5702 Niederlenz (CH)
(74) Vertreter: Frei, Alexandra Sarah
(86) Internationale Anmeldenummer: PCT/CH2002/000132
(87) Internationale Veröffentlichungsnummer: WO 2002/069817

(56) Entgegenhaltungen:
- EP-A- 0 617 935
- WO-A-98/42988
- GB-A- 2 277 448
- US-A- 3 919 775
- US-A- 4 566 138
- DATABASE WPI Section PQ, Week 198313 Derwent Publications Ltd., London, GB; Class P31, AN 1983-E3008K XP002200302 & SU 929 072 A (OMSK.MED.INST UND OMSK.POLY.)

## Beschreibung

Die Erfindung betrifft ein Implantat nach dem Oberbegriff des ersten unabhängigen Patentanspruchs. Das Implantat dient zur Erstellung von mindestens teilweise formschlüssigen Verbindungen mit menschlichen oder tierischen Gewebeteilen, insbesondere mit Skelettteilen, wobei mit Hilfe der Implantate Gewebeteile miteinander oder Gewebeteile mit künstlichen, beispielsweise Gewebeteile stützenden oder ersetzenden Mitteln oder anderen therapeutischen Hilfsvorrichtungen verbunden werden. Ferner betrifft die Erfindung eine Vorrichtung nach dem Oberbegriff des entsprechenden, unabhängigen Patentanspruchs. Die Vorrichtung und ein Verfahren dienen zur Implantation der genannten Implantate.

Bekannte Implantate zur Erstellung von Verbindungen mit Skelettteilen (Knochen) sind beispielsweise Schrauben, Stifte, Agraffen etc., mit denen Knochen mit Knochen oder Kochen mit künstlichen, tragenden, stabilisierenden, stützenden oder Skelettteile ersetzenden Teilen (Stabilisierungs- oder Fixierungsplatten, Fäden, Drähte, künstliche Gelenkelemente, künstliche Zähne, etc.) verbunden werden. Solche zu implantierende Verbindungselemente bestehen beispielsweise aus Metall oder Kunststoff, auch aus resorbierbarem Kunststoff. Sie werden nach einer Heilung gegebenenfalls operativ wieder entfernt, sie können aber auch im Körper belassen werden, wo sie gegebenenfalls allmählich abgebaut und durch vitales Gewebe ersetzt werden.

Zur Stabilisierung eines Knochenbruches wird beispielsweise eine Fixierungsplatte mit entsprechenden Löchern mittels der genannten Schrauben im Bereiche der Bruchstelle fixiert. Platte und Schrauben bestehen dabei beispielsweise aus Metall (z.B. rostfreier Stahl oder Titan). Die Schrauben sind selbstschneidend und werden in gewindelose Öffnungen im Knochen gedreht oder sie werden in vorgebohrte Gewinde-Öffnungen eingeschraubt. Für ähnliche Zwecke werden auch Stifte und Agraffen in vorher erstellte Öffnungen eingeschlagen. Die erstellten Verbindungen sind in der Regel reibschlüssig, gegebenenfalls auch formschlüssig.

In allen Fällen sind beim Einsetzen der genannten Verbindungselemente relativ hohe Kräfte (Torsionskräfte-und Schlagkräfte) aufzuwenden, für wieder zu entfernende derartige Implantate auch bei ihrer Entfernung. Dies bedeutet vielfach, dass die Implantate für das Implantieren und gegebenenfalls Entfernen eine höhere mechanische Stabilität aufweisen müssen, als für die Last, die sie in implantiertem Zustand zu tragen haben. Insbesondere für Implantate aus resorbierbaren Kunststoffen mit einer bedeutend kleineren mechanischen Festigkeit als Metall, führt dies dazu, dass die Implantate relativ grosse Querschnitte aufzuweisen haben und deshalb für die Implantation auch unerwünscht grosse Öffnungen im vitalen Gewebe erstellt werden müssen.

Das mechanische Einbringen der Implantate, bei dem beispielsweise die den Reibschluss erzeugende Reibung überwunden werden muss, ist auch mit der Entwicklung von erheblichen, das umliegende Gewebe beeinträchtigenden Wärmemengen verbunden, insbesondere das Schneiden von Gewinden, das Einschrauben von selbstschneidenden Schrauben und das Einschlagen von Implantaten ohne Vorbohrung. Zur Erstellung der genannten Verbindungen sind in der Chirurgie auch aushärtbare, plastische Materialien (z.B. partikuläre Zemente auf hydraulischer oder polymerer Basis) bekannt, die in einem hochviskosen Zustand von aussen zwischen Implantate und vitales Gewebe oder in Gewebedefekte eingepresst und in situ ausgehärtet werden. Damit sind auch formschlüssige Verbindungen erstellbar, allerdings nur, wenn die Öffnungen, in die solche Materialien eingepresst werden, entsprechende Hinterschneidungen aufweisen.

Aus der Publikation US-3919775 ist es ferner bekannt, Dichtungspfropfen, die aus Guttapercha bestehen, in den Kanal einer Zahnwurzel einzubringen, um diesen abzudichten. Der Pfropfen wird mit Hilfe eines Instrumentes in den Kanal gepresst, wobei das Instrument gegebenenfalls elektrisch beheizt oder mit Ultraschall angeregt wird, um den Pfropfen zusätzlich zu erweichen, damit er möglichst vollständig in den Wurzelkanal und in die Seitenkanäle gepresst werden kann.

Es ist nun die Aufgabe der Erfindung, Implantate zur Erstellung von formschlüssigen Verbindungen mit Gewebeteilen (insbesondere Knochen-, Knorpel-, Bänder-, Sehnen-Teile, aber auch andere Gewebeteile) zu schaffen, welche Implantate einfach, mit kleinen Kräften und insbesondere schnell implantierbar sind und sofort (Primärstabilität) sehr gute Verbindungen liefern können. Zusätzlich wäre es wünschbar, wenn die Implantate bezüglich Wärmemengen und Spannungskonzentrationen weniger Probleme ergeben, als dies mindestens für einen Teil der bekannten Implantate der Fall ist, und wenn das Volumen des zu implantierenden Fremdmaterials weiter reduziert werden könnte. Ferner ist es die Aufgabe der Erfindung, eine Vorrichtung und ein Verfahren zum Implantieren der Implantate zu schaffen.

Diese Aufgaben werden gelöst durch die Implantate und die Vorrichtung, wie sie in den Patentansprüchen definiert sind.

Die Erfindung macht sich die an sich, z.B. aus der Publikation WO-98/42988, bekannte Tatsache zu nutzen, dass insbesondere thermoplastische Polymermaterialien durch Anwendung von mechanischen Schwingungen gezielt verflüssigt und in diesem Zustand durch hydrostatischen Druck in Hohlräume (z.B. Poren von Holz) eingepresst werden können, wodurch nach dem Erstarren formschlüssige Verbindungen entstehen.

Gemäss Erfindung bestehen die für die Erstellung von formschlüssigen Verbindungen mit Gewebeteilen dienenden Implantate also mindestens teilweise aus einem Material, das bei relativ tiefen Temperaturen (<250°C) durch mechanische Schwingungs-Energie (insbesondere Ultraschall), das heisst durch innere und/oder äussere Reibung in einen derart verflüssigten Zustand bringbar sind, dass sie unter der Wirkung eines äusseren Druckes in Poren oder andere Öffnungen des Gewebeteils pressbar sind, wo sie, wieder erstarrt, den Formschluss bilden.

Als durch mechanische Energie verflüssigbare Materialien für die erfindungsgemässen Implantate eignen sich im wesentlichen bei relativ niedrigen Temperaturen plastisch werdende Polymere, insbesondere die medizinisch bereits verwendeten Thermoplasten Polyethylen (PE), Polymethylmetakrylat (PMME), Polycarbonat (PC), Polyamide, Polyester, Polyacrylate oder entsprechende Copolymere, die nicht resorbierbar sind, oder die resorbierbaren Polymere auf Milch- und/oder Glykolsäurebasis (PLA, PLLA, PGA, PLGA etc.), sowie Polyhydroxyalkanoate (PHA), Polycaprolactone (PCL), Polysaccharide, Polydioxanone (PD), Polyanhydride oder entsprechende Copolymere. Ebenfalls geeignet sind die an sich bekannten, hydraulischen oder polymeren Zemente, die thixotrope Eigenschaften haben, wie beispielsweise Calziumphosphat-, Calziumsulfat- oder Methacrylat-Zemente, die auch thixotrop aufgearbeitete, körpereigene oder transplantierte Materialien enthalten können. Solche Zemente können dank ihrer thixotropen Eigenschaften mit mechanischer Energie (insbesondere Ultraschall) ohne Temperaturerhöhung von einem hochviskosen Zustand in einen flüssigen Zustand gebracht werden.

Zur Implantation wird ein erfindungsgemässes Implantat mit dem Gewebeteil (auf der Oberfläche oder in einer für das Implantat gegebenenfalls speziell erstellten Öffnung) in Kontakt gebracht und dann mit z.B. Ultraschallenergie beaufschlagt und dabei gegen den Gewebeteil gedrückt. Durch entsprechende Formgebung des Implantates und durch entsprechende Dosierung der Energie kann dafür gesorgt werden, dass von dem verflüssigbaren Material gezielt örtlich nur ein benötigtes Minimum verflüssigt wird. Wenn genügend Material verflüssigt und eingepresst ist, wird die Energiezufuhr gestoppt, so dass das verflüssigte Material in seiner neuen Form erstarrt, wobei der Druck auf das Implantat vorzugsweise aufrechterhalten wird.

Für die Implantation werden die genannten Materialien also nicht durch externe Wärme, sondern mittels mechanischer Energie (Schwingungsenergie, Vibrationsenergie) infolge innerer und/oder äusserer Reibung verflüssigt. Dabei bleibt die thermische Belastung des umliegenden Gewebes gering. Durch die mechanische Energie wird eine hohe Scherwirkung zwischen unterschiedlichen Materialphasen erreicht. Dies trägt dazu bei, dass die zu verflüssigenden Materialien bei geringer Belastung der Umgebung gleichmässig verflüssigt werden und eine niedrige Viskosität erreichen. Das derart verflüssigte Material wird dann mittels hydrostatischem Druck in Poren oder Öffnungen das umliegenden Gewebes gepresst. So ist es möglich, das umliegende Gewebe unter Ausnützung dieses hydrostatischen Effekts verstärkend zu durchsetzen.

Bei Bedarf ist es vorteilhaft, dem verflüssigbaren Material für weitere Funktionen zusätzliche Stoffe beizugeben, beispielsweise Stoffe, die das Material mechanisch verstärken, die es in einer sekundären Reaktion aufquellen lassen oder darin Poren bilden, Stoffe, die in die vitale Umgebung zur Förderung einer Heilung oder Regeneration freigesetzt werden sollen, oder Stoffe, die andere Funktionen übernehmen sollen. Als heilungs- oder regenerationsfördemde Stoffe können dem Material beispielsweise Wachstumsfaktoren, Antibiotika oder Entzündungshemmer beigegeben werden, derart, dass diese Stoffe mit dem Materialfluss gezielt an eine gewünschte Stelle einbringbar, bzw. in einem Gewebebereich verteilbar und im Falle eines resorbierbaren Materials verzögert freigebbar sind.

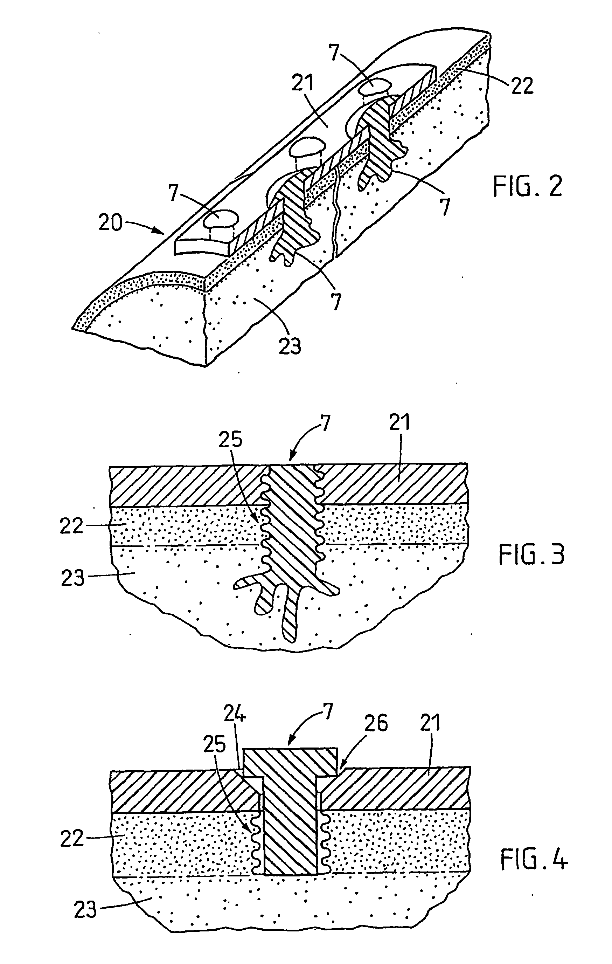

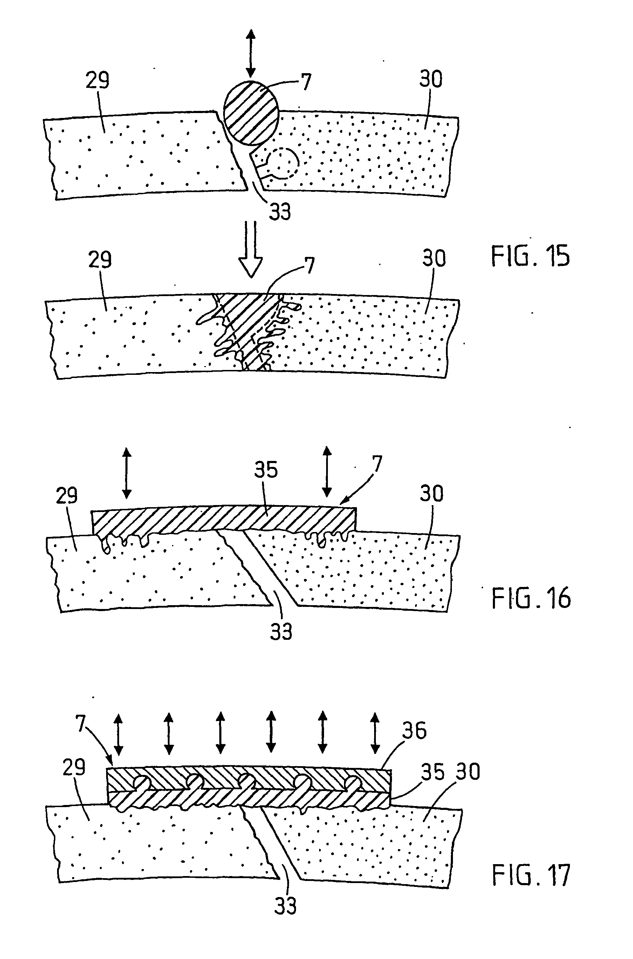

Mit erfindungsgemässen Verbindungs-Implantaten sind mehr punktuelle oder grösserflächige Verbindungen möglich. Die Lastverteilung kann dadurch gezielt beeinflusst und gesteuert werden. Bspw. können mittels erfindungsgemässen Implantaten Fixierungs- oder Stabilisierungsplatten grossflächig an einer Knochen-Oberfläche (siehe z.B. Figuren 15 oder 16) oder aber mehr punktuell und tiefenwirksam (siehe Figuren 2 bis 4) befestigt werden. Verbindungen an der Oberfläche werden z.B. erreicht indem Platten oder andere Stütz- oder Fixiervorrichtungen, die integrierte Verflüssigungszonen oder ganze Verflüssigungsschichten aufweisen, auf zu verbindende Stellen gelegt und anschliessend mindestens örtlich zur Verflüssigung mit mechanischer Energie (z.B. Ultraschall-Vibration) angeregt werden. Die verflüssigbaren Bereiche sind dabei vorteilhafterweise mit Energierichtungsgebern ausgestattet oder stehen mit solchen in Kontakt. Diese Energierichtungsgeber unterstützen eine lokale Verflüssigung. Bei Energierichtungsgebem kann es sich um vorstehende Elemente handeln, die z.B. pyramiden-, kegel-, halbkugel- oder rippenförmig ausgestaltet sind und eine Konzentration der aufgebrachten mechanischen Energie bewirken.

Tiefenwirksame Verankerungen werden erzielt, indem beispielsweise stift- oder dübelförmige Implantate, die einen über ihre Länge gleichbleibenden oder sich ändernden Querschnitt (oder Querschnittsgeometrie) aufweisen und die ganz oder teilweise aus einem verflüssigbaren Material bestehen, auf die Gewebeoberfläche aufgebracht oder in diese eingebracht und dann angeregt werden. Diese Implantate sind mit Vorteil so ausgestaltet, dass die Verflüssigung an vorgegebenen Stellen (Spitze, spezifische Schaftbereiche) einsetzt. Eine kontrollierte Verflüssigung kann auch hier mittels Energierichtungsgebern (vorstehende, definiert geformte Elemente) erreicht werden. Durch Einbringen des Implantates in die Oberfläche wird eine tiefenwirksame Verankerung erzielt. Die hydrostatischen Verhältnisse können so gewählt werden, dass das flüssige Material unter grossem Druck in die angrenzende Umgebung gepresst wird.

Die für die Implantation der erfindungsgemässen Implantate, das heisst für die Verflüssigung des verflüssigbaren Materials eines mit dem Gewebeteil in Kontakt stehenden Implantates und für dessen Einpressung in das Gewebe verwendete Vorrichtung kann zusätzlich für eine aktive Kontrolle der Temperaturverteilung im umliegenden Gewebe und Material dienen, so dass untragbare Wärmemengen und Temperaturen und dadurch bedingte Beeinträchtigungen von vitalem Gewebe wirksam verhindert werden. Der Implantationsprozess wird gegebenenfalls durch eine aktive Steuerung der eingebrachten und abgeführten Energie (Wärmeverteilung und Management) mittels Steuerung der Vorrichtung und gegebenenfalls geeignet angebrachten Sensoren und Wärmeleitelementen gesteuert. Das heisst, durch Dosierung der eingebrachten Energie und Abführen von überschüssiger Energie wird Einfluss genommen.

Die zur Materialverflüssigung eingesetzte Energie wird bevorzugt mittels piezoelektrischer oder magnetorestriktiver Anregung erzeugt. Eine Schwingeinheit (z.B. Piezoelement plus Sonotrode), die über einen Generator angesteuert zum Schwingen angeregt wird, überträgt Wellen im Frequenzbereich von etwa 2 bis 200 kHz, vorzugsweise Ultraschall (z.B. 20 oder 40 kHz) auf das damit wirkverbundene (dagegen gepresste) Implantat. Dieses ist mit dem zu verbindenden Knochen, resp. Gewebe derart gekoppelt, dass die Schallenergie in einer inneren oder oberflächlichen Energieabsorbtion des verflüssigbaren Materials resultiert, wodurch dieses mindestens örtlich verflüssigt wird. Der Verflüssigungsprozess wird durch eine hohe Scherwirkung erzielt. Durch eine zweite Komponente mit unterschiedlicher Dichte, die im zu verflüssigenden Material lokal (z.B. als Kügelchen) eingelagert ist, kann die innere Reibung und damit eine innere Verflüssigung beeinflusst werden, wie dies z.B. bei Verwendung von thixotropen, partikulären Zementen als Implantat oder Implantatteil der Fall ist.

Die durch das mit den erfindungsgemäß Implanten und Vorrichtung aus zu führende Verfahren erzeugten Verbindungen beruhen vornehmlich auf Formschluss, wobei die Formschlussmittel beidseitig sehr klein (Oberflächenunebenheiten oder Oberflächenrauhigkeit, Gewebeporen) oder auch grösser (grössere Hohlräume im Gewebe oder zwischen Gewebeteilen oder mechanisch erstellte Öffnungen oder Hohlräume im Gewebe) sein können. Die Verbindungs-Implantate werden mittels Ultraschall mechanisch angeregt, derart, dass sie kontrolliert insbesondere im Kontaktbereich mit dem Gewebeteil oder in ihrem Innern verflüssigt werden. Die Verflüssigung findet in der Regel an der Oberfläche des Gewebeteils oder in einer entsprechenden Vertiefung (Öffnung) statt, die z.B. durch das Eindringen des Verbindungs-Implantates durch die Oberfläche ins Gewebe entstanden ist oder vorgängig mit anderen mechanischen Mitteln erstellt wurde.

Ein zu beobachtender Effekt beim tiefenwirksamen Einbringen von verflüssigbarem Material kann stark vereinfacht und schematisch wie folgt beschrieben werden: Ähnlich einem Kolben in einem hydraulischen Zylinder, sitzt noch nicht verflüssigtes Material des Verbindungs-Implantates in einer Öffnung und füllt diese im wesentlichen dichtend aus. Da das verflüssigte Material deshalb nicht aus der Öffnung entweichen kann, bildet sich aufgrund der von aussen wirkenden Last (Druck auf das Implantat) ein hydrostatischer Druck. Dieser und die Ultraschallschwingungen führen dazu, dass verflüssigtes Material in bestehende und/oder neu gebildete Hohlräume des umliegenden zu verbindenden Materials (vitales Gewebe) gepresst wird. Die Eindringtiefe ist dabei u.a. abhängig von der Beschaffenheit dieser Umgebung, von den eingestellten Betriebsparametern und vom verflüssigbaren Material (insbesondere von seiner Viskosität). Durch das verflüssigbare oder verflüssigte Volumen des Verbindungs-Implantates kann die Menge des ins Gewebe gepressten Materials bestimmt werden. Falls viel Material notwendig sein sollte, oder die Grösse und die Anzahl der im Gewebe vorhandenen Hohlräume nicht bekannt sein sollte, ist es möglich, Implantate oder Implantaskomponenten zu verwenden die beliebig nachführbar sind.

Durch das verdrängte und komprimierte Material erzeugte Spannungsspitzen, die zum Versagen, z.B. Sprengen, des Gewebes führen könnten, werden vermieden durch die gezielte und aufeinander abgestimmte Applikation von Ultraschall und mechanischem bzw. hydrostatischem Druck, sowie durch eine entsprechende Ausgestaltung der Implantate und der daran angeordneten, verflüssigbaren Materialien. Hohlräume und Spalten werden durch das verflüssigte Material gefüllt, bei genügend porösem Gewebe gegebenenfalls ohne Vorbohren. Durch eine kontrollierte, bereichsweise Materialkompression in angrenzenden Gewebezonen wird erreicht, dass Verbindungs-Implantate starken Rückhalt selbst in stark porösen Geweben (z.B. osteoporotischem Knochengewebe) finden. Dadurch werden hohe mechanische Auszugskräfte bzw. Belastbarkeiten erreicht, die sich bei Verwendung von mindestens teilweise resorbierbarem Material nach einer Anfangsphase eines Heilungsprozesses kontrolliert reduzieren, bzw. durch nachwachsendes Gewebe übernommen werden (sekundäre Stabilisierung).

Die Erfindung eignet sich beispielsweise zur Verankerung einer Zahnprothese in einem Kiefer. Die Zahnprothese weist dabei vorzugsweise einen normierten Unterbau auf, der als erfindungsgemässes .Implantat ausgebildet ist und der mit verschiedenen Zahnaufsätzen verbindbar ist. Der Unterbau besteht also ganz oder teilweise aus durch mechanische Energie verflüssigbarem Material, das eingesetzt in eine bestehende Öffnung im Kiefer durch Anregen mittels mechanischer Energie verflüssigt und in Poren des Knochengewebes eingepresst wird. Das Implantat passt sich dabei in die Öffnung und in die Gewebeporen ein. Dadurch entsteht eine sofort stabile (primäre Stabilisierung), nicht nur in der Zahnwurzelöffnung im Kiefer sondern auch im benachbarten Knochengewebe gut verankerte Basis für das Befestigen eines Zahnoberteils, die gegebenenfalls später mindestens teilweise von regeneriertem Knochengewebe ersetzt wird (sekundäre Stabilisierung).

Ein weiterer Anwendungsbereich der hier offenbarten Erfindung besteht im Bereich der Implantationsmedizin von z.B. künstlichen Gelenkelementen. Sowohl eine künstliche Gelenkpfanne als auch eine Gelenkkugel bzw. deren Schaft können mittels erfindungsgemässen Implantaten mit dem vitalen Knochengewebe verbunden bzw. darin verankert werden. Zusätzlich zur schonenden Lasteinleitung bei der Implantation werden die beteiligten Materialien so gewählt, dass Steifigkeitssprünge weitgehend vermieden werden, was alles positiv zur Lebensdauer des Implantats beiträgt.

Die für die Implantation der erfindungsgemässen Implantate verwendete Vorrichtung kann vereinfacht wie folgt beschrieben werden: Ein Generator dient zum Erzeugen von elektrischen Schwingungen, die über ein Übertragungsmittel, z.B. ein Kabel, auf eine Schwingeinheit übertragen werden. Die Schwingeinheit weist typischer Weise ein Schwingelement (Antriebseinheit) und einen Resonator auf, die miteinander wirkverbunden sind. Die Antriebseinheit (z.B. Piezoelement) regt den Resonator zum Schwingen an. So eine Vorrichtung ist aus GB-A-2277448 bekannt, auf dessen Offenbarung der Oberbegriff des Anspruchs 17 basiert. Die Schwingungen des Resonators werden unmittelbar oder über ein Übertragungsmittel auf das Implantat übertragen. Dies verflüssigt sich dabei mindestens örtlich infolge innerer Verflüssigung oder infolge eines Kontakts mit einer nicht schwingenden Umgebung (Gewebeteiloder anderer Implantatsteil). Das Implantat kann dabei mittels einer entsprechenden Halterung gehalten werden und wird mittels einer Führung geführt. Insbesondere für minimal invasive Chirurgie eignet sich eine Befestigung des Implantates direkt an der Schwingeinheit. Besonders günstig sind Halterungs- und/oder Führungs-Vorrichtungen die nicht nur einem temporären Halten oder Fixieren des Implantates sondern auch dem Temperaturmanagement (insbesondere Wärmeabfuhr) dienen.

Aufgrund der Art und Weise, wie das Material des Implantates gezielt und örtlich verflüssigt wird, werden keine grossen Wärmemengen erzeugt. Zusätzlich kann die Temperatur der dem Implantat benachbarten Gewebebereiche mittels Tempera-turmanagement aktiv kontrolliert werden, beispielsweise mittels Wärmeleitelementen, die gezielt wärmeabführend wirken oder mit Kühlflüssigkeiten (z.B. sterile Ringerlösung), die temperaturkontrollierend wirken.

Das Verfahren zur Implantation eines erfindungsgemässen Implantates z.B. am menschlichen und tierischen Skelett wird wie folgt durchgeführt: das Implantat wird mit dem Skelettteil in Kontakt gebracht, dann werden mechanische Schwingungen erzeugt und auf das durch mechanische Energie verflüssigbare Material des Implantates übertragen, während das Implantat gegen den Skelettteil gepresst wird. Mechanische Energie wird zugeführt, solange, bis das verflüssigbare Material genügend verflüssigt ist und im Bereiche des Kontaktes in das Knochengewebe oder mindestes in Oberflächenunebenheiten des Skeletteils eindringt. Dann werden die mechanischen Schwingungen gestoppt, wodurch das verflüssigte Material erstarrt (vorteilhafterweise unter Aufrechterhaltung des hydrostatischen Druckes) und das Implantat im Skeletteil formschlüssig verankert.

Erfindungsgemässe Verbindungs-Implantate haben bspw. die Form von Stiften, Dübeln oder von Platten/Folien. Sie dienen zur Verbindung von Gewebeteilen untereinander oder von Gewebeteilen mit künstlichen Elementen.

Vorteilhafterweise wird für Implantationen der oben beschriebenen Art ein Kit oder Set verwendet, das mindestens einen Typus von erfindungsgemässen Implantaten, vorteilhafterweise eine Auswahl von verschieden dimensionierten, für einen Anwendungsbereich geeigneten Implantaten umfasst sowie eine Vorrichtung zur Durchführung der Implantation. Vorteilhafterweise umfasst das Kit auch Mittel zum sterilen Gebrauch der Vorrichtung (sterile Umhüllungen für die Vorrichtung) und gegebenenfalls Austauschstücke von Bestandteilen (insbesondere Resonator, distaler Resonatorteil oder Übertragungsteil), die sterilisierbar sind. Diese Resonatorteile können auch durch verschiedene Formen an verschiedene Implantate und/oder Anwendungen angepasst sein. Weiter weist das Kit vorteilhafterweise Anleitungen zur Implantation, Angaben über Implantationsparameter für spezifische Implantatstypen und weitere Hilfsmittel zur Vorbereitung des Gewebeteiles (z.B. auf die Implantate abgestimmte Bohrer), Positionierungsinstrumente, Kontrollinstrumente oder an Implantate und/oder Resonatorten angepasste Implantatsführungen auf.

Das Kit oder Set wird vorzugsweise durch Nachlieferung von Implantaten stets verfügungsbereit gehalten. Die Auswahl richtet sich nach den Anforderungen und kann sich mit der Zeit ändern. Die Nachlieferungen (Ersatz- und Zusatzkit) umfassen einerseits Ersatz für verbrauchte Implantate und neue Implantatstypen, wiederum beispielsweise mit entsprechenden Mitteln zur Gewebevorbereitung, Positionierungsinstrumenten, Kontrollinstrumenten, angepassten Resonatoren oder Resonatorteilen, Implantatsführungen und insbesondere entsprechende Implantationsanweisungen.

Die Erfindung wird anhand der nachfolgenden Figuren näher erläutert. Dabei zeigen:
- **Figur 1**: eine beispielhafte Ausführungsform der Vorrichtung zur Implantation von erfindungsgemässen Implantaten und ihre Verwendung;
- **Figur 2**: eine mit erfindungsgemässen Implantaten an einem Knochen befestigte Fixierungsplatte;
- **Figuren 3 und 4**: Beispiele von erfindungsgemässen Implantaten, die beispielsweise in der Anwendung gemäss Figur 2 verwendbar sind, und damit erstellte Verbindungen zwischen Knochen und Platte;
- **Figuren 5a bis 5e**: beispielhafte Querschnitte von stiftförmigen, erfindungsgemässen Implantaten, die achsial verlaufende Energierichtungsgeber aufweisen;
- **Figuren 6 bis 8**: Längsschnitte durch zwei beispielhafte, stiftförmige, erfindungsgemässe Implantate mit Implantatsteilen aus einem nicht verflüssigbaren Material;
- **Figuren 9 bis 13**: beispielhafte Ausführungsformen von kooperierenden Haltemitteln an stift-oder dübelförmigen Implantaten und Resonatoren;
- **Figur 14**: Anwendungen von erfindungsgemässen Implantaten im Bereiche von menschlichen Schädel- und Kieferknochen;
- **Figuren 15 bis 17**: beispielsweise im Schädelbereich anwendbare erfindungsgemässe Implantate und damit erstellte, beispielhafte Verbindungen zwischen zwei Schädelknochenteilen;
- **Figur 18**: eine beispielhafte Resonatoranordnung für die Anwendungen nach Figuren 16 und 17;
- **Figur 19**: eine weitere Anwendung von erfindungsgemässen Implantaten im Bereiche der menschlichen Wirbelsäule;
- **Figur 20**: eine weitere Anwendung von erfindungsgemässen Implantaten zur Fixierung eines Zahnersatzes;
- **Figuren 21 und 22**: zwei beispielhafte, erfindungsgemässe Implantate für die Anwendung gemäss Figur 20 im Schnitt;
- **Figur 23**: eine Fixiervorrichtung, die mit erfindungsgemässen Implantaten an einem Unterarmknochen befestigt ist;
- **Figur 24**: ein Beispiel eines für die Anwendung gemäss Figur 23 geeigneten Verbindungs-Implantates;
- **Figur 25**: die mit Implantaten gemäss Figur 24 am Knochen befestigte Fixiervorrichtung gemäss Figur 23 im Schnitt;
- **Figur 26**: ein weiteres Beispiel eines für die Anwendung gemäss Figur 23 geeigneten Verbindungs-Implantates;
- **Figur 27**: eine mit Hilfe eines erfindungsgemässen Implantates fixierte Trochanterplatte zur Fixierung eines gebrochenen Gelenkhalses;
- **Figur 28**: ein mit einem erfindungsgemässen Implantat an einem Röhrenknochen befestigter Schaft für eine künstliche Gelenkkugel;
- **Figur 29**: ein mit erfindungsgemässen Implantaten an Knochen befestigtes Gelenkband;
- **Figur 30**: einen Schnitt durch einen mit einem Implantat auszufüllenden und ausgefüllten, beispielsweise von einem Tumor erzeugten Gewebehohlraum.

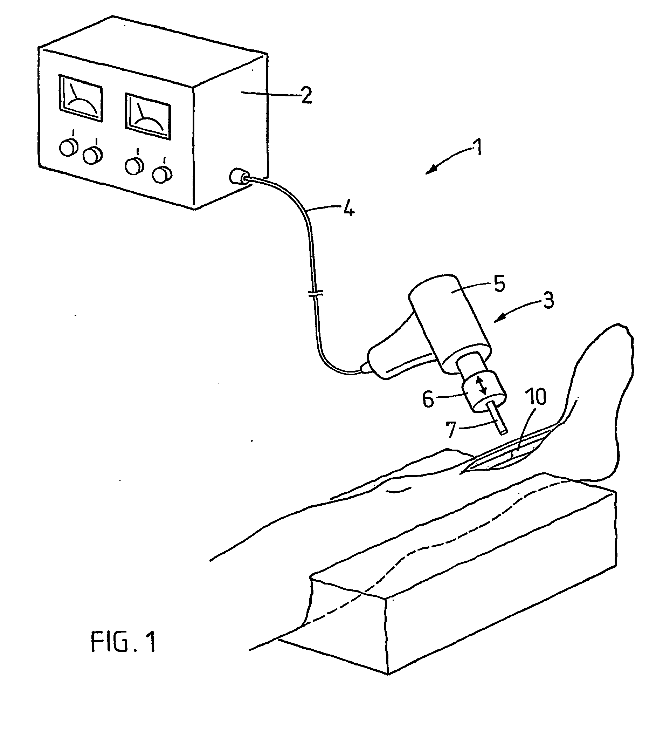

**Figur 1** zeigt schematisch und stark vereinfacht eine beispielhafte Ausführungsform einer Implantationsvorrichtung 1, die zum Implantieren von erfindungsgemässen Implantaten anwendbar ist.

Die Vorrichtung 1 weist einen Generator 2 und eine Schwingeinheit 3 auf die über ein Kabel 4 miteinander verbunden sind. Die teilweise in einem Gehäuse 5 untergebrachte Schwingeinheit 3 ist als Handgerät ausgestaltet, das von der Handhabung her ähnlich wie eine Handbohrmaschine verwendet wird. Die Schwingeinheit 3 weist ein im Gehäuse 5 integriertes Schwingelement (nicht näher dargestellt) auf, das mit einem Resonator (Sonotrode) 6 wirkverbunden ist. Mindestens ein distaler Resonatorteil ragt aus dem Gehäuse 5. Der Generator 2 versorgt das Schwingelement mit Energie. Durch das Schwingelement angeregt schwingt der Resonator mit einer vorgegebenen Frequenz oder gegebenenfalls mit einem vorgegebenen Frequenzmuster. Besonders geeignet sind Frequenzen von 2 bis 200 kHz und Resonatoramplituden von 1 bis 100µm, in der durch den Doppelpfeil angedeuteten Richtung (z-Richtung). Die Frequenzen können abhängig vom Anwendungsgebiet, von den zu verflüssigenden Materialien und von der Form von Resonator und Implantat einstellbar sein. Es ist auch denkbar, den Vibrationen im Ultraschallbereich zusätzliche mechanische Schwingungen z.B. mit niedriger Frequenz und grösserer Amplitude zu überlagem. In vielen Fällen wird es aber genügen, wenn die Vorrichtung für eine einzige Schwingfrequenz ausgelegt ist, beispielsweise für 20 oder 40 kHz und beispielsweise für eine Resonatoramplitude von ca. 20 bis 30µm in z-Richtung (Richtung, in der ein Implantat 7 durch den Resonator 6 gegen einen Gewebeteil gedrückt wird). Zur Regelung der Leistung (zugeführte Energie pro Zeiteinheit) kann die Anregung pulsiert sein, wobei Pulsabstände und gegebenenfalls Pulslängen einstellbar sind.

Vorteilhafterweise wird in an sich bekannter Art die Schwingfrequenz und die Resonatorform derart aufeinander abgestimmt, dass der Resonator in einer stehenden Welle schwingt und sein distales Ende, mit dem er auf das Implantat gedrückt wird, eine maximale Amplitude in z-Richtung aufweist. Ferner ist es vorteilhaft, stiftförmigen Implantaten eine Länge zu geben, die auf eine vorgegebene Anregerfrequenz und ein vorgegebenes Implantatmaterial abgestimmt ist.

Das distale Ende des Resonators 6 kann wie in der Figur 1 dargestellt zur Halterung eines Implantates 7 ausgebildet sein, was die Positionierung des Implantates an einem Gewebeteil oder in einer Öffnung eines Gewebeteils, hier eines Beinknochens 10, erleichtert. Für eine Positionierung und Implantation ohne Öffnung, ist eine Implantatsführung vorzusehen, die am Gehäuse 5 abgestützt ist. Es ist aber durchaus auch möglich in einer Ausführung der nicht Teil der Erfindung ist, den Resonator 6 im Sinne eines Hammers mit einer planen Stirnfläche auszugestalten und einfach gegen ein in einer Gewebeöffnung oder durch eine geeignete, separate Halterung oder Führung gehaltenes Implantat zu drücken. Für einen derartigen Resonator ist dafür zu sorgen, dass seine distale Stirnfläche während der Implantation nicht mit dem Implantat verklebt. Dies wird beispielsweise erreicht durch eine entsprechende, nicht haftende Stirnfläche des Resonators oder durch einen an den Resonator grenzenden Implantatteil, der aus einem nicht verflüssigbaren Material besteht.

Damit die Vorrichtung im sterilen Operationsbereich anwendbar ist, wird sie beispielsweise in einer sterilen Umhüllung angewendet. Vorteilhafterweise weist die sterile Hülle für den distalen Teil des Resonators eine Öffnung auf und ist der Resonator oder der distale Teil des Resonators abnehmbar, austauschbar und sterilisierbar.

Andere, beispielhafte Ausführungsformen der erfindungsgemässen Implantationsvorrichtung 1 sind beispielsweise als Handgeräte, die alle Bestandteile (inkl. Energieversorgung z.B. als Akku) aufweisen, ausgebildet oder auch als stationäre Geräte.

**Figur 2** zeigt einen durch erfindungsgemässe Implantate 7 im Bereiche eines Knochenbruches oder -risses an einem Knochenteil 20 befestigte Fixierungs- oder Stabilisierungsplatte 21, mit deren Hilfe der Riss oder Bruch stabilisiert wird. Der Knochenteil 20 weist in diesem Falle eine äussere Kortikalis-Schicht 22 auf, die nicht sehr dick aber relativ kompakt ist, und darunter Spongiosa-Gewebe 23, das porös ist. Anders als in der Figur 2 dargestellt, ist der Übergang von Kortikalis zu Spongiosa im natürlichen Gewebe ein allmählicher Übergang, in dem das Gewebe immer poröser wird. Die Implantate 7 reichen durch die Platte 21, die dafür entsprechende Öffnungen aufweist, durch die Kortikalis 22 und in die Spongiosa 23 und sind mindestens in der Spongiosa 23 verankert.

**Figuren 3 und 4** zeigen im Schnitt und in einem grösserem Massstab zwei Beispiele von erfindungsgemässen Implantaten 7, die beispielsweise für die in der Figur 2 dargestellte Anwendung verwendbar sind. Figur 3 zeigt ein Implantat nach der Implantation, Figur 4 ein anderes Implantat, das für die Beaufschlagung mit Schwingungs-Energie in einer Öffnung 24 von Platte 21 und Kortikalis 22 positioniert ist.

Für die Implantation ist mindestens die Kortikalis-Schicht 22 beispielsweise durch Bohren zu öffnen. Eine entsprechende Bohrung kann sich gegebenenfalls als Sackloch in die Spongiosa 23 fortsetzen. Da die Kortikalis keine für das Einpressen von verflüssigtem Material geeignete Poren aufweist, können in ihr beispielsweise durch Schneiden eines Gewindes 25 oder durch Aufrauhen der Bohrungs-Innenwände Öffnungen oder Oberflächenrauheiten geschaffen werden, in die das verflüssigte Material eingepresst wird und unter Bildung einer formschlüssigen Verbindung erstarrt. In der Spongiosa 23 wird das verflüssigte Material des Implantates in die Poren eingepresst und derart das Implantat 7 tiefenwirksam verankert. Es zeigt sich, dass das hydrostatische Einpressen des flüssigen Materials in die Gewebeporen bedeutend Gewebe-schonender ist als ein mechanisches Einbringen eines festen Materials. Aus diesem Grunde können auch stabile Verbindungen mit Geweben ohne grosse mechanische Festigkeit wie beispielsweise mit osteoporotischem Knochengewebe erstellt werden.

Zur Verbindung des Implantates 7 mit der Platte 21 kann wie für mechanische Schrauben ein Kopf vorgesehen werden, wie dies in der Figur 2 dargestellt ist. Andererseits kann auch gemäss Figur 3 in der Platte 21, die beispielsweise aus Metall besteht, wie in der Kortikalis 22 ein Gewinde vorgesehen werden, in das verflüssigtes Material eindringt und unter Bildung eines Formschlusses erstarrt. In diesem Falle kann auf einen Kopf verzichtet werden. Das Implantat 7 wird mit der Platte 21 fluchtend ausgerichtet nicht durch unerwünschtes Trimmen eines überstehenden Teils sondern durch Eintreiben eines entsprechend dimensionierten Implantates bis in die gewünschte Stellung.

Für eine aus einem thermoplastischen Kunststoff bestehende Platte 21, wie sie in der Figur 4 dargestellt ist, kann die Verbindung zwischen Platte und Implantat (Lockerungssicherung) durch eine stoffschlüssige Verbindung (Verschweissung oder Klebung) gleichzeitig mit der Verankerung im Gewebe erstellt werden. Diese stoffschlüssige Verbindung beginnt sich beim Eintreiben des Implantates an der Verbindungsstelle 26 zu bilden. Auch in diesem Falle wird das Implantat 7 vorteilhafterweise so weit eingetrieben, dass es am Schluss mit der Aussenseite der Platte 21 fluchtet.

Da die Implantate 7 nicht in das Gewebe eingedreht werden müssen, müssen sie nicht wie mechanisch einbringbare Schrauben mit Mitteln ausgestaltet werden, die zur Einkoppelung einer relativ grossen Torsionskraft ausgelegt sind. Die Dimensionierung der Implantate kann rein auf ihre Funktion im implantierten Zustand ausgerichtet werden, das heisst, die Implantate können schlanker und die im Gewebe zu erstellenden Öffnungen kleiner ausgelegt werden als dies für herkömmliche Schrauben aus demselben Material der Fall ist. Da die formschlüssigen Formen über eine Verflüssigung und ein Wiedererstarren von Material entstehen, sind sie ärmer an Spannungen und Kerbungen, was ihre Festigkeit weiter erhöht und ihnen eine kleinere Anfälligkeit auf Materialermüdung verleiht.

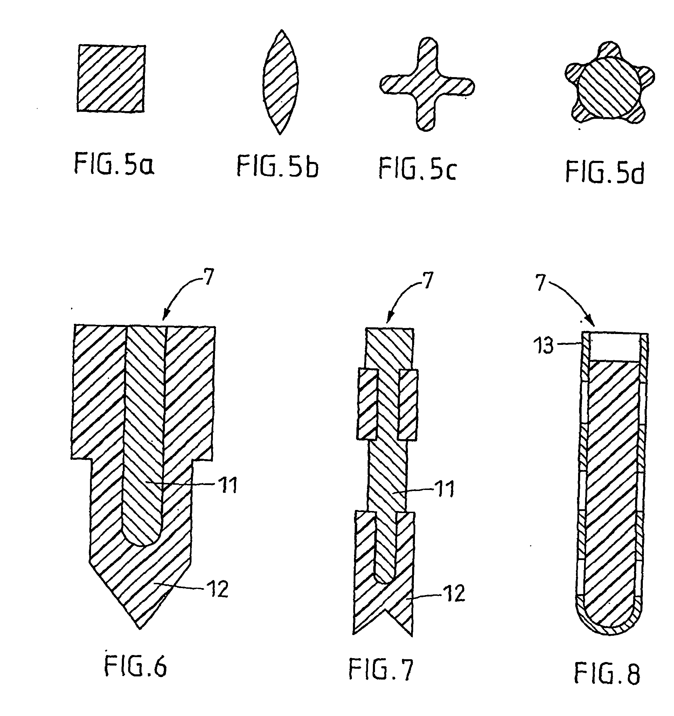

Erfindungsgemässe Implantate, die wie in den Figuren 2 bis 4 tiefenwirksam im Gewebeteil zu verankern sind, sind vorteilhafterweise stift- oder dübelförmig und weisen das verflüssigbare Material beispielsweise an ihrem distalen Ende auf sowie an weiteren Oberflächenbereichen, an denen eine Verankerung wünschenswert ist (z.B. in Gewinde in Platte 21 und Kortikalis 22). Sie können dazu, wie in den Figuren 2 bis 4 dargestellt vollständig aus dem verflüssigbaren Material bestehen, wobei das distale Ende und Oberflächenbereiche, an denen das Material insbesondere zu verflüssigen ist, vorteilhafterweise mit Energierichtungsgebern ausgestattet sind, oder an mit diesen Bereichen in Kontakt stehenden Oberflächen derartige Energierichtungsgeber vorgesehen werden. Derartige Energierichtungsgeber sind beispielsweise distale Implantatsenden, die als Spitze oder in einer anderen Art sich zu einem oder mehreren, im wesentlichen punkt- oder linienförmigen Spitzenbereichen verjüngend ausgebildet sind. Zu verflüssigende weitere Oberflächenbereiche weisen beispielsweise Höcker, Spitzen oder Rippen auf, deren Höhen und Breiten an die an die zu erstellende Verankerung anzupassen sind. Die Energierichtungsgeber ragen mindestens 10µm über die Oberfläche, können aber auch bedeutend grösser sein und beispielsweise als axial verlaufende Rippen zu höckerigen oder kantigen Stiftquerschnitten führen, wie sie beispielhaft in den **Figuren 5a bis 5e** dargestellt sind. Stiftförmige Implantate weisen derartige Querschnitte über ihre ganze Länge oder nur über einen Teil ihrer Länge auf.

Für stiftförmige Implantate, die zusätzlich zu einer Verankerung im Bereiche ihres distalen Endes oder als alleinige Verankerung im Bereiche ihrer Mantelfläche zu verankern sind, sind Gewebeöffnungen (z.B. Bohrungen) vorzusehen, die beim Einführen des Implantates mindestens stellenweise zu einer Reibpassung zwischen Gewebe und Implantat bzw. Energierichtungsgebem führen, die also ein wenig enger sind als der Querschnitt der Implantate.

Das verflüssigbare Material kann für weitere Funktionen Fremdphasen oder weitere Stoffe enthalten. Insbesondere kann das verflüssigbare Material durch Beimischung von Fasern oder Whiskern (z.B. Calziumphosphat-Keramiken oder -Gläser) mechanisch verstärkt sein, kann das verflüssigbare Material in situ quellende oder lösliche und dadurch porenbildende Bestandteile (z.B. Polyester, Polysaccharide, Hydrogele, Natriumphosphat) aufweisen oder können ihm in situ freizusetzende Stoffe wie Wachstumsfaktoren, Antibiotika, Entzündungshemmer oder Puffer (z.B. Natriumphosphat) gegen die nachteiligen Wirkungen sauren Abbaus beigegeben werden. Auch Beimischungen zur Sichtbarmachung in Röntgenbildern und ähnliche Funktionen sind denkbar.

Es zeigt sich, dass zur Verankerung in Spngiosa von Implantaten gemäss Figuren 2 bis 4, die aus Polymeren wie beispielsweise PC oder PLLA bestehen und einen Durchmesser von 3 bis 4mm haben, vorteilhafterweise für das Einpressen Kräfte im Bereiche von 0.5 bis 5 N pro mm² Implantatsquerschnitt angewendet werden. Mit Kräften der genannten Grössenordnung können die Implantate mit einer Geschwindigkeit, die höher ist als 5 mm/s, in das Gewebe getrieben werden.

**Figuren 6 bis 8** zeigen drei weitere, beispielhafte stiftförmige Implantate 7. Die zusätzlich zu Bereichen aus verflüssigbarem Material einen Kern 11 (Figuren 6 und 7) oder eine Hülse 13 (Figur 8) aus einem nicht verflüssigbaren Material, beispielsweise aus Metall, Keramik, Glas oder aus einem Verbundwerkstoff auf.

Die Implantate gemäss Figuren 6 und 7 weisen an ihrem distalen Ende eine Kappe 12 aus dem verflüssigbaren Material auf, die mehr oder weniger zugespitzt ist (Figur 6) oder mehrere spitz oder linienförmig zusammenlaufende Endbereiche (Figur 7) aufweist. Die Mantelfläche des Kerns 11 ist vollständig vom verflüssigbaren Material umgeben (Figur 6) oder nur bereichsweise, wobei diese Bereiche sich axial erstrecken, ringförmig (Figur 7) sein können oder regelmässig oder unregelmässig über die Mantelfläche verteilt sein können. Auch an diesen Mantelflächenbereichen sind vorteilhafterweise Energierichtungsgeber vorzusehen, wie sie weiter ober für ganz aus verflüssigbarem Material bestehende Implantate beschrieben sind. Je nach gewünschter Verankerungstiefe im Mantelbereich ist das verflüssigbare Material dicker oder dünner, es soll aber nicht dünner sein als ca. 10µm.

Als Energierichtungsgeber sind auch stufenförmige Querschnittsreduktionen, wie dies in der Figur 6 dargestellt ist, geeignet. Implantate mit derartigen Stufen werden vorteilhafterweise in entsprechend gestufte oder sich verengende Gewebeöffnungen implantiert.

Die Beaufschlagung eines stift- oder dübelförmigen Implantates mit nicht verflüssigbarem Kern 11 kann entweder das ganze proximale Implantatsende betreffen oder nur den aus dem verflüssigbaren Material bestehenden, ringförmigen Aussenbereich.

Das Implantat gemäss Figur 8 weist das verflüssigbare Material im Innern der nicht verflüssigbaren Hülse 13 auf. Die Hülse 13 ist mit Öffnungen versehen, die da angeordnet sind, wo eine Verankerung erwünscht ist. Eine derartige Ausführungsform des erfindungsgemässen Implantates ist insbesondere geeignet für die Anwendung von hochviskosen, thixotropen Materialien als verflüssigbares Material, da ein derartiges Material nicht in der Lage ist, der mechanischen Belastung des auf das Implantat drückenden Resonators standzuhalten. Die Öffnungen in der Hülse sind derart zu dimensionieren, dass das hochviskose Material nicht austreten kann, wohl aber die verflüssigte Form davon. Insbesondere geeignet sind Hülsen 13 aus porösen Sintermaterialien. Ein Implantat mit Hülse 13 ist in einer Gewebeöffnung zu positionieren und der Resonator ist nur auf das verflüssigbare Material aufzusetzen, also in seinem Querschnitt an den inneren Querschnitt der Hülse anzupassen.

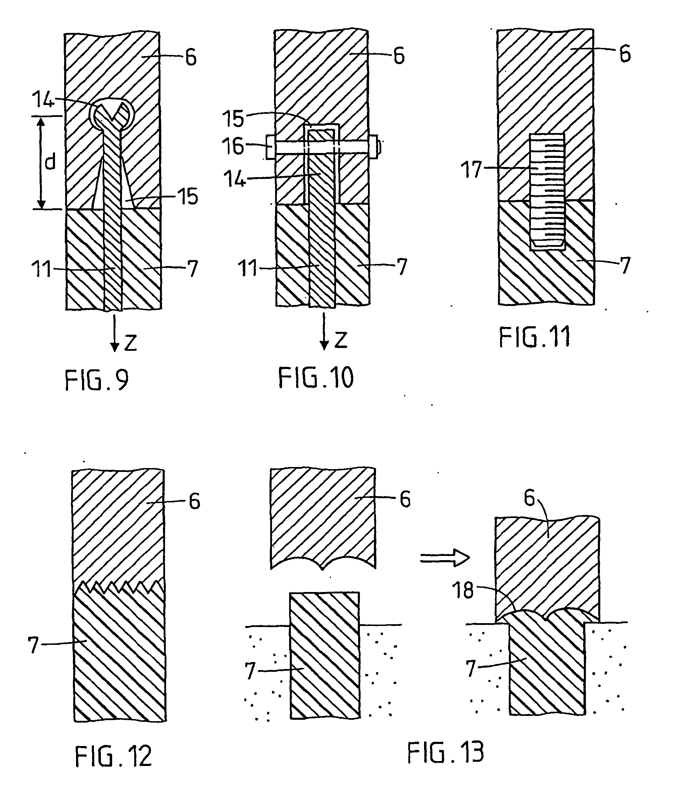

Am proximalen Ende von stift- oder dübelförmigen Implantaten kann beispielsweise eine kopfartige Verdickung vorgesehen sein, kann ein einen Gewebeteil ersetzender oder fixierender, künstlicher Teil oder eine andere, therapeutische Hilfsvorrichtung befestigt oder befestigbar sein oder kann ein Fixiermittel für einen Nahtfaden oder einen Cerclage-Draht angeordnet sein. Am proximalen Ende eines stift- oder dübelförmigen Implantates kann auch ein Haltemittel angeordnet sein, das mit einem entsprechenden Haltemittel am Resonator kooperiert (sieh Figuren 9 bis 11).

Ein beispielsweise metallischer Kern 11 in einem mittels Ultraschall zu implantierenden, stift- oder dübelförmigen Implantat dient üblicherweise als mechanische Verstärkung des Implantates und ist dafür entsprechend dimensioniert. Der Kern kann aber auch bedeutend dünner und leicht aus dem Implantat entfembar sein. Er dient in diesem Falle beispielsweise als Sichtbarmacher im Röntgenbild z.B. für eine minimal invasive Implantation oder als Führungsdraht und wird unmittelbar nach der Implantation wieder entfernt.

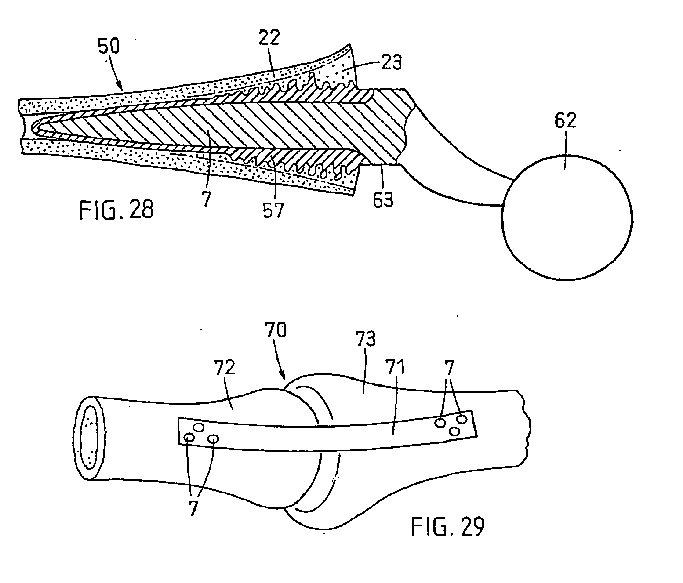

Einem Implantat mit z.B. metallenem Kern, das erfindungsgemäss mit einem verflüssigbaren und resorbierbaren Material im Gewebe verankert wird, gibt dieses Material eine sofortige, primäre Stabilität. Durch den Abbau des Verankerungsmaterials lockert sich die Verankerung und eine durch das Implantat erzeugte Stabilisierung wird dynamisiert, so dass sukzessive mehr Kräfte vom Gewebe selber zu tragen sind, was einen natürlichen Regenerationsprozess fördert und Abbauprozesse in vielen Fällen verhindert. Der Kern kann nach dem Abbau des verflüssigbaren Materials sehr leicht entfernt werden, wenn seine Oberfläche derart ausgestaltet ist, dass das vitale Gewebe nicht damit verwächst. Wenn seine Oberfläche aber derart ausgestaltet ist, dass eine Verwachsung gefördert wird (bioaktive Oberfläche), ergibt sich eine ideale, sekundäre Stabilität für ein im Gewebe zu verbleibendes Implantat bzw. für einen im Gewebe zu verbleibenden Implantatskern (siehe auch Figur 28).

Implantatskerne, wie sie in den Figuren 6 und 7 dargestellt sind, können nicht nur aus Metall (z.B. Stähle, Titan oder Cobalt-Chrom-Legierungen) sondern je nach Anwendung auch aus Kunststoffen (z.B. Polyetherarylketone, Polyfluor- und/oder - chlorethylene, Polyetherimide, Polyethersulfone, Polyvinylchlorid, Polyurethane, Polysulfone, Polyester) oder aus keramischen oder glasartigen Materialien (z.B. Aluminiumoxid, Zirkonoxid, Silikate, Calziumphosphat-Keramiken oder -Gläser) oder aus Verbundwerkstoffen (z.B. Kohlefaser-verstärkte Hochtemperatur-Thermoplaste) bestehen.

**Figuren 9 bis 13** zeigen verschiedene beispielhafte Anwendungen zur Halterung eines erfindungsgemässen, stift- oder dübelförmigen Implantates 7 im oder am distalen Teil des Resonators 6 (Sonotrode) der Implantationsvorrichtung 1 (Figur 1). Diese Halterung kann beispielsweise eine formschlüssige Halterung sein, wie dies in den Figuren 9 und 10 dargestellt ist. Der Formschluss ist beispielsweise realisiert als Schnappverschluss (Figur 9) einer federnd ausgestalteten proximalen Verlängerung 14 eines Implantatkerns 11 oder Implantates 7, die in eine entsprechende Öffnung 15 am distalen Ende des Resonators 6 einführbar ist und dort einschnappt. Der Formschluss kann auch realisiert werden mit Hilfe eines durch Resonator 6 und proximale Verlängerung 14 eines Implantatkerns 11 oder Implantats 7 reichenden und entsprechend gesicherten Stiftes 16. Dabei wird vorteilhafterweise dafür gesorgt, dass der Formschluss in einer Distanz d zum distalen Ende des Resonators angeordnet ist, in der für Schwingungen in z-Richtung ein Knotenpunkt liegt, die Amplitude in z-Richtung also im wesentlichen gleich Null ist.

Figur 11 zeigt eine kraftschlüssige Verbindung zwischen Resonator.6 und Implantat 7 mittels Verschraubung 17. Wenn diese Verschraubung derart vorgespannt ist, dass die Schwingungen sich ungebrochen über die Grenzfläche fortpflanzen, stellt das Implantat 7 einen Teil des Resonators 6 dar und ist entsprechend auszulegen. Das heisst, das distale Ende des Resonators 6 braucht keine maximale Amplitude in z-Richtung aufzuweisen, es kann hier auch beispielsweise ein Knotenpunkt liegen.

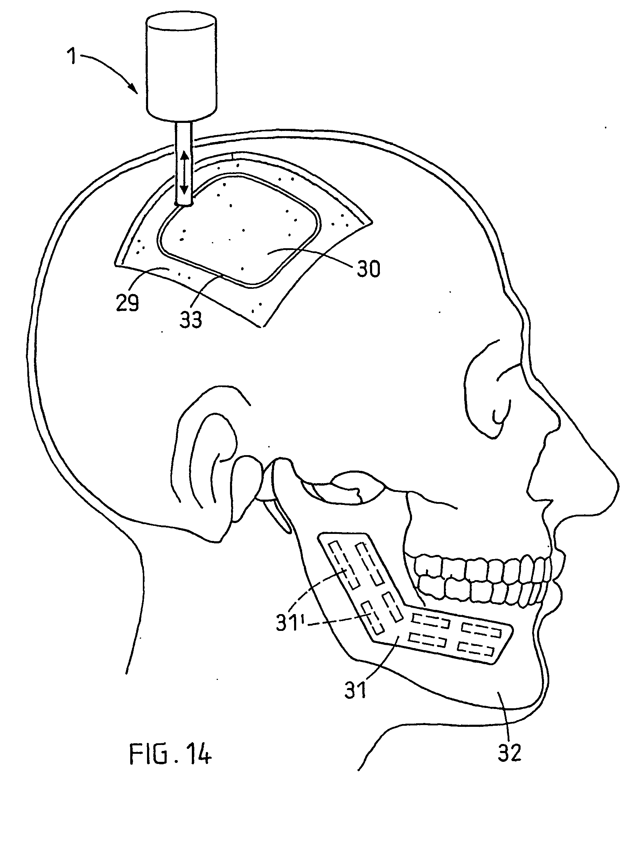

Figuren 12 und 13 zeigen vorteilhafte Halterungen von Implantaten 7 am Resonator 6 für Implantate, deren proximales Ende aus dem verflüssigbaren Material bestehen. Es handelt sich in beiden Fällen um eine durch die Ultraschall-Einwirkung bedingte Formgebung und Verklebung des proximalen Endes des Implantats 7 am distalen Ende des Resonators 6, die durch entsprechende Energierichtungsgeber am Resonator 6 initiiert werden. Figur 12 zeigt einen Resonator 6 mit einer distalen Fläche, die wie die Schlagfläche eines Stockhammers ausgebildet ist, Figur 12 einen Resonator 6 mit einem zentralen Energierichtungsgeber. In beiden Fällen wird das proximale Ende des Implantates 7 mit den Energierichtungsgebem des Resonators 6 kontaktiert und der Resonator 6 in Schwingung versetzt. Das verflüssigbare Material im Bereiche der Energierichtungsgeber des Resonators wird als Erstes verflüssigt und verklebt mit dem Resonator, wobei es die Form dessen distaler Fläche annimmt und im Falle, der in der Figur 12 dargestellt ist, einen Kopf 18 bildet.

Die Halterung der Implantate, wie sie in den Figuren 9 bis 13 dargestellt sind, werden vorteilhafterweise vor der Positionierung des Implantates auf dem oder im Gewebeteil erstellt und sie werden nach der Implantation wieder gelöst, in den Fällen gemäss Figuren 12 und 13 durch eine Kraft mit der der Resonator 6 vom Implantat 7 gelöst, z.B. abgeknickt oder abgedreht wird.

**Figur 14** zeigt als weitere Anwendungsbereiche für erfindungsgemässe Implantate die Fixierung einer in eine Öffnung der Schädeldecke 29 eingepasste Deckplatte 30 aus Knochen oder aus einem künstlichen Material und die Fixierung einer beispielsweise künstlichen Fixierungsplatte 31 auf einem gebrochenen oder gerissenen Kieferknochen 32. Gleiche Anwendungen sind in der Wiederherstellungschirurgie im Gesichtsbereich denkbar. Die zwischen der Deckplatte 30 und dem umgebenden Knochengewebe herzustellenden Verbindungen beschränken sich vorteilhafterweise auf ausgewählte Stellen der Spalte 33 zwischen Platte und nativem Knochen. Ebenso wird die Fixierungsplatte 31 an ausgewählten Plattenstellen 31' mit dem Kieferknochen verbunden. Die Verbindungen an den ausgewählten Stellen werden durch aufeinanderfolgende Implantationsschritte mit Hilfe der Implantationsvorrichtung 1 erstellt.

**Figuren 15 bis 17** zeigen im Schnitt und in einem grösseren Massstab mit erfindungsgemässen Implantaten 7 erstellbare Verbindungen, die sich beispielsweise für die in der Figur 14 dargestellten Anwendungen eignen.

**Figur 15** zeigt ein erfindungsgemässes Implantat 7, das für eine mindestens örtliche Verbindung zwischen dem Schädelknochen 29 und der in eine Öffnung des Schädelknochens einzusetzenden Deckplatte 30, die beispielsweise aus einem porösen Material (z.B. ebenfalls Schädelknochen) besteht, positioniert (oben) und dann mittels Ultraschallenergie (Doppelpfeil) implantiert wird, um Schädelknochen 29 und Deckplatte 30 über die Spalte 33 zu verbinden (unten).

Die Spalte 33 ist vorteilhafterweise schief ausgestaltet, derart, dass Druckkräfte von aussen auf den Spaltenbereich durch die Schädeldecke 29 aufgenommen werden, und auf der Aussenseite ist die Spalte 33 für die Positionierung des Implantates 7 erweitert. Das Implantat, das beispielsweise kugel- oder wurstförmig ist und aus einem thermoplastischen oder thixotropen Material besteht, wird in der äusseren Spaltenerweiterung positioniert und mit der Schwingungsenergie beaufschlagt. Dadurch wird das Implantatmaterial verflüssigt und auf der einen Seite in die Poren der Schädeldecke 29 und auf der anderen Seite in entsprechende Poren der aus Knochen bestehenden Deckplatte 30 oder in entsprechend angeordnete, künstlich erstellte Öffnungen (z.B. strichpunktierte Nut) in einer künstlichen Platte eingepresst. So entsteht eine in beiden Seiten formschlüssig verankerte Verbindung zwischen Schädeldecke 29 und Deckplatte 30.

**Figur 16** zeigt eine Fixierungsfolie 35, die auch die Form eines textilen Flächengebildes haben kann und die beispielsweise zur örtlichen Fixierung der Deckplatte 30 in der Öffnung des Schädelknochens 29 verwendbar ist. Die Folie 35 ist beispielsweise bandförmig und vorteilhafterweise flexibel. Sie besteht ganz aus einem verflüssigbaren Thermoplasten oder ist beispielsweise mit einer Fasermatte oder einer ähnlichen Struktur verstärkt. Sie wird über die Spalte 33 gelegt und beidseitig (Doppelpfeile) mit Hilfe der Implantationsvorrichtung (Figur 1) angeregt, derart, dass sie mit der Oberfläche der Schädeldecke 29 und der Deckplatte 30 verklebt (grösserflächige, wenig tiefenwirksame Verbindung, die sich auch auf eine Vielzahl oder ein Muster von einzelnen Fixierungspunkten beschränken kann). Gegebenenfalls werden die Oberflächenbereiche, an denen das Implantat mit dem darunter liegenden Material verbunden werden soll, entsprechend vorbehandelt (z.B. aufgerauht) oder sind an der künstlichen Platte 30 entsprechende Oberflächenstrukturen (Oberflächenunebenheiten, Vertiefungen, Rillen etc.) vorgesehen. Für die Verbindung der Folie 35 mit einer Knochenoberfläche ist ein Druck in der Grössenordnung von 0,5 bis 3 N pro mm² Resonatorstimfläche genügend.

**Figur 17** zeigt. eine Fixierungsplatte 36, die mit Hilfe einer Fixierungsfolie 35 oder entsprechendem, textilem Flächengebilde über der Spalte 33 befestigt ist und die für die Aufnahme von entsprechend grösseren Kräften beispielsweise aus Metall besteht und deshalb neben der Schädelanwendung auch in der ebenfalls in der Figur 13 dargestellten Kieferanwendung oder in der Anwendung gemäss Figur 2 verwendet werden kann. Die Fixierungsplatte 36 besteht also aus einem im Sinne der Erfindung nicht verflüssigbaren Material und weist an ihrer gegen das zu fixierende Gewebe gerichteten Oberfläche eine für einen Formschluss geeignete Oberflächenstruktur auf. Die Folie 35 wird zwischen Platte 36 und zu fixierendem Gewebe bzw. Material positioniert und durch die Platte 36 mindestens lokal mit Schwingungsenergie beaufschlagt und dadurch mit der Oberfläche der Schädeldecke 29 bzw. der Deckplatte 30 verbunden. Die formschlüssige Verbindung zwischen Folie 35 und Fixierungsplatte 36 kann während der Implantation erstellt werden oder die Platte 36 kann mit der damit verbundenen Folie 35 als fertiges Implantat verwendet werden. In einem derartigen, zweischichtigen Implantat kann die Verbindung zwischen den Schichten auch eine stoffschlüssige sein (Verklebung oder Verschweissung). Die Folie 35 kann sich in einem derartigen, zweischichtigen Implantat auch auf eine Beschichtung der Platte beschränken, welche Beschichtung vorteilhafterweise nicht eine konstante Dicke hat sondern im Sinne von Energierichtungsgebern aus einem Muster von Hökkern, Spitzen oder Rippen besteht, die eine minimale Höhe (Beschichtungsdicke) von ca. 10µm aufweisen.

Die in der Figur 14 dargestellte Fixierungsplatte 31 weist beispielsweise in entsprechenden Vertiefungen angeordnete Folienbereiche 31' mit einer mit Energierichtungsgebem versehenen Aussenoberfläche auf und wird an diesen Stellen mit dem darunterliegenden Kieferknochen verbunden.

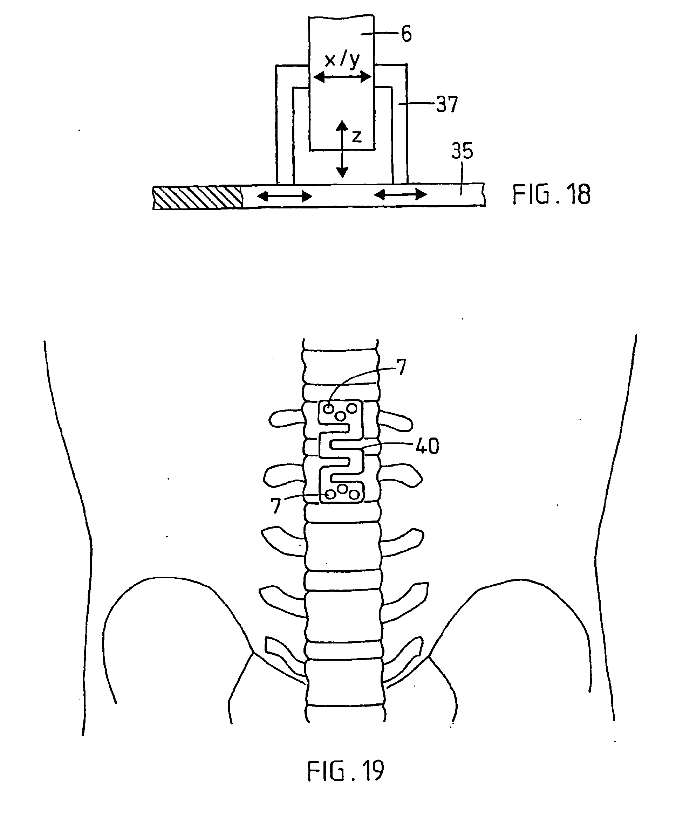

Insbesondere für die in der Figur 16 dargestellte Anwendung mag es vorteilhaft sein, den zu verwendenden Resonator derart auszugestalten, dass die auf das Implantat übertragenen Schwingungen nicht, wie in den Figuren mit Doppelpfeilen angedeutet, senkrecht zur zu erstellenden Verbindungsfläche (z-Richtung) ausgerichtet sind sondern parallel dazu (x/y-Richtung). Dazu eignet sich gegebenenfalls ein Übertragungselement 37, wie es in der **Figur 18** dargestellt ist. Dieses Übertragungselement 37 ist mit dem Resonator 6 kraftschlüssig verbunden und zwar an einer Stelle, in der die Welle in z-Richtung einen Knotenpunkt (Amplitude = 0) und deshalb die Welle in x/y-Richtung eine maximale Amplitude aufweist. Diese Schwingung in x/y-Richtung wird durch das Übertragungselement 37 auf die Folie 35 übertragen.

**Figur 19** zeigt schematisch und stark vereinfacht als weitere Anwendung von erfindungsgemässen Implantaten ein Stützelement 40 für einen menschlichen Wirbelsäulenbereich. Das Stützelement 40 ist elastisch und stützt den Wirbelsäulenbereich stationär oder gegebenenfalls vorübergehend. Das Stützelement 40 wird im Sinne der Erfindung an Wirbelkörpern befestigt, dadurch, dass es selbst aus einem entsprechend verflüssigbaren Material besteht und ohne Tiefenwirkung befestigt wird (analog zur Verbindung gemäss Figur 16), dadurch, dass es aus einem nicht verflüssigbaren Material besteht und mittels Folie und ohne Tiefenwirkung (analog Figur 17) oder mit Vorbohren und Tiefenwirkung (analog Figuren 2 bis 4) mit den Wirbelkörpem verbunden wird. In der Figur dargestellt sind stiftförmige Implantate 7, die beispielsweise einen das Stützelement überstehenden Kopf aufweisen, der gemäss Figur 12 hergestellt ist. Für eine stationäre Stützung werden verbindende Implantate und Stützelement aus nicht resorbierbaren Materialien verwendet, für eine vorübergehende Stützung sind Teile aus resorbierbaren Materialien denkbar.

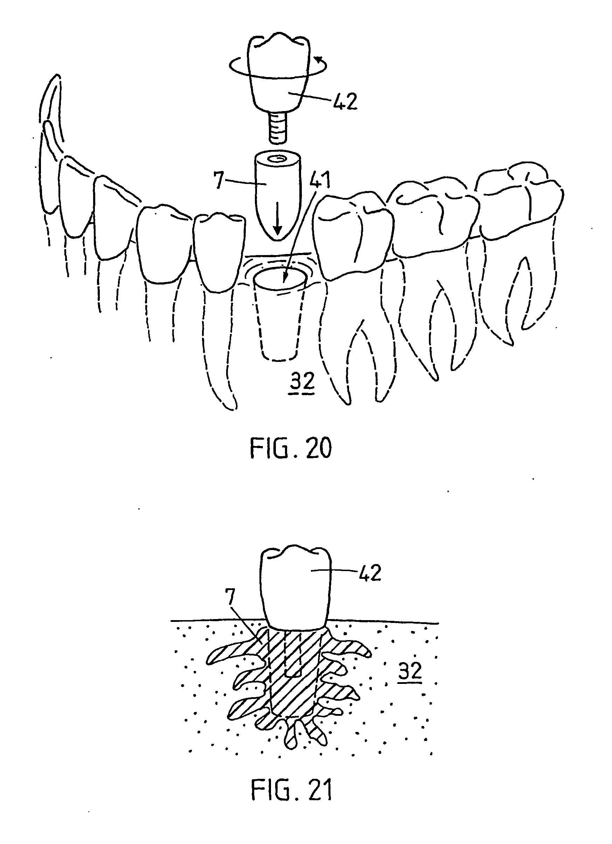

**Figur 20** zeigt die Anwendung eines dübelförmigen, erfindungsgemässen Implantates 7 als Basis für einen künstlichen Zahn 40 in einem Kieferknochen 32. Das Implantat 7 besteht mindestens teilweise aus einem thermoplastischen oder thixotropen Material. In der Stirnseite weist es Mittel zur Halterung des künstlichen Zahns 40, einer Brücke oder einer Prothese auf. Das Implantat wird mit oder ohne den künstlichen Zahn in die entsprechende Öffnung 41 eingesetzt und unter Ultraschall-Vibration weiter hineingedrückt. Da sich dabei mindestens ein Teil des Implantates verflüssigt, wird es nicht nur die Öffnung weitgehend lückenlos ausfüllen sondern auch in die Poren des Kieferknochens eingepresst werden, so dass eine tiefenwirksame Verbindung entsteht, wie sie beispielsweise in der **Figur 21** im Schnitt dargestellt ist.

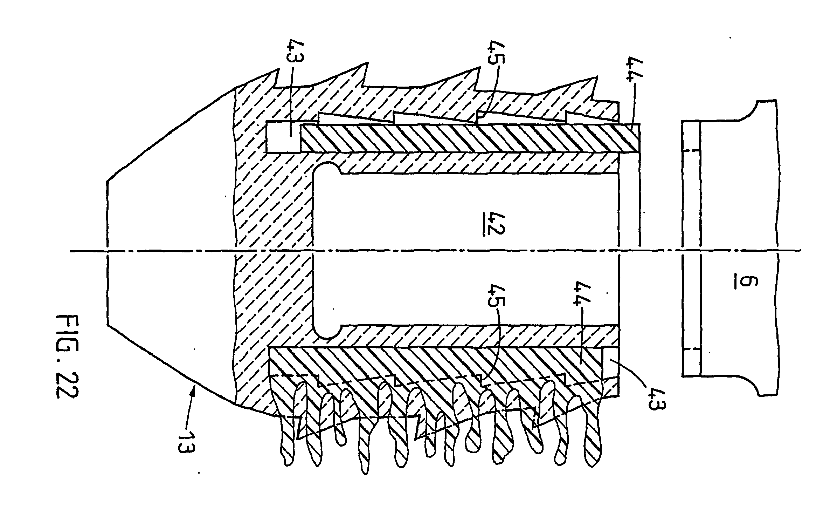

**Figur 22** zeigt im Schnitt eine weitere, beispielhafte Ausführungsform des erfindungsgemässen Implantates, die sich insbesondere für die in der Figur 20 dargestellte Anwendung eignet. Es handelt sich hier um ein Implantat, dessen Bereich aus verflüssigbarem Material nicht an seine Oberfläche angeordnet ist, sondern in einer für das verflüssigbare Material in seinem verflüssigten Zustand durchlässigen Hülse 13, wie dies bereits im Zusammenhang mit der Figur 8 beschrieben wurde. Das Implantat ist längs geschnitten dargestellt und links von der Mittellinie vor der Anwendung des Ultraschalls und rechts von der Mittellinie nach der Anwendung des Ultraschalls gezeigt. Die Hülse 13 besteht aus einem beispielsweise metallischen oder keramischen Sintermaterial mit einer offenen Porosität und übernimmt die tragende Funktion des Implantats. Sie weist im dargestellten Falle eine Öffnung 42 auf, die beispielsweise mit einem Innengewinde versehen ist und in der ein künstlicher Zahn, eine Brücke oder Zahnprothese befestigbar ist. Sie weist eine weitere, Ringförmige Öffnung 43 auf, in der das verflüssigbare Material, beispielsweise ein zylinderförmiges Stück 44 aus dem verflüssigbaren Material positioniert ist. Für eine gezielte Verflüssigung sind im Innern der ringförmigen Öffnung 43 Energierichtungsgeber 45 angeordnet, mit denen das verflüssigbare Material in Kontakt steht.

Das Implantat gemäss Figur 22 wird beispielsweise in einer Öffnung eines Kieferknochens (41, Figur 20) positioniert und dann wird mit Hilfe eines in seinem distalen Bereich ringförmig ausgestalteten Resonators 6 das verflüssigbare Material mit mechanischer Energie beaufschlagt. Das Material wird dadurch verflüssigt und durch das poröse Hülsenmaterial in das umliegende Knochengewebe gepresst, wobei das Implantat in diesem Gewebe verankert wird.

Für die in den Figuren 19 bis 20 dargestellte Anwendung ist es insbesondere vorteilhaft, das als verflüssigbares Material ein resorbierbares Material zu wählen, während, während der tragend Teil aus einem weder verflüssigbaren und nicht resorbierbaren Material besteht, das eine genügende mechanische Festigkeit für die Befestigung des Zahns, der Brücke oder Prothese aufweist. Dabei ist mindestens die Oberfläche des zentralen Teils bioaktiv (z.B. porös wie für die Hülse 13 beschrieben), das heisst derart, dass sie ein Zusammenwachsen mit Knochengewebe fördert. Ein derartiges Implantat weist sofort nach der Implantation eine Primärstabilität auf, die für Befestigung und normalen Gebrauch von Zahn, Brücke oder Prothese ausreicht. Vitales Material ersetzt dann sukzessive das resorbierbare Material und wächst dann mit dem zentralen Implantatteil zusammen, was durch dessen bioaktive Oberfläche gefördert wird. Das erfindungsgemässe Implantat bietet also eine sofortige Primärstabilität ohne die Verwendung von Zement und nach der Resorptions- und Regenerationsphase eine dauerhafte, sekundäre Stabilität, die der Stabilität bekannter Implantate ebenbürtig ist. Im Vergleich zu bekannten Implantationsverfahren fällt aber eine Übergangsphase weg, in der gemäss Stand der Technik die Öffnung 41 geschlossen wird und die Regeneration des Knochengewebes abgewartet wird, bevor der Zahn, die Brücke oder die Prothese direkt im regenerierten Knochen befestigt wird.

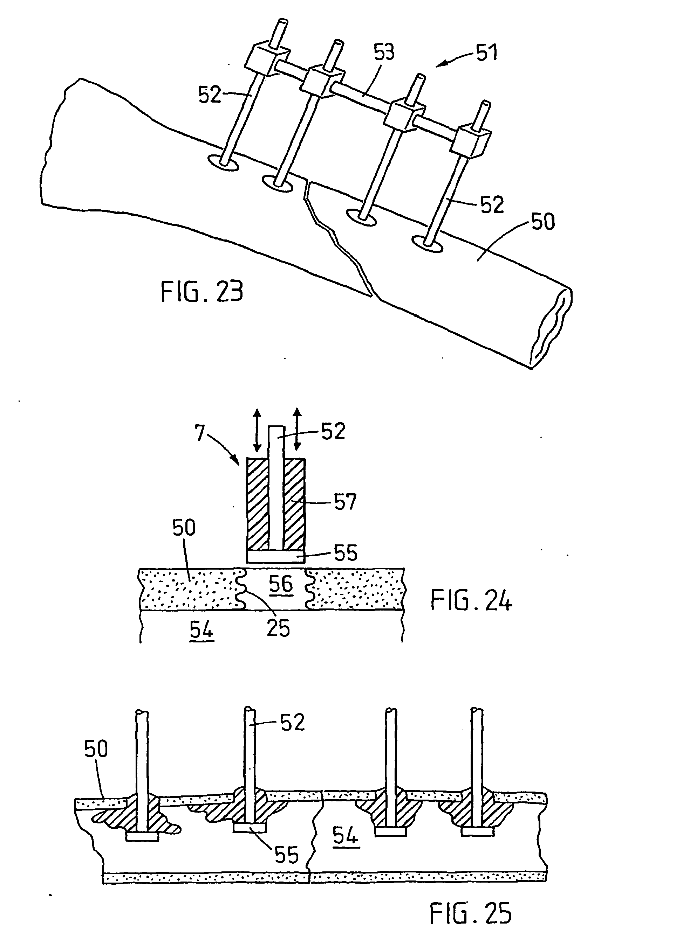

**Figur 23** zeigt eine beispielsweise an einem Röhrenknochen 50 eines menschlichen Armes mittels erfindungsgemässen Implantaten befestigte, externe Fixiervorrichtung 51 mit Stützen 52 und einem an den Stützen 52 befestigten Träger 53. Die Stützen 52 sind dabei als erfindungsgemässe Implantate ausgerüstet. Der mediale Teil eines Röhrenknochens weist mehrheitlich Kortikalis auf und nur sehr wenig im Sinne der Erfindung poröse Gewebebereiche. Aus diesem Grunde wird, wie in den Figuren 24 und 25 in mehr Detail gezeigt, der Markraum 54 im Inneren des Röhrenknochens 50 ausgenützt, um das verflüssigte Material hinein zu pressen. Da das Mark aber dem hydrostatischen Druck nur einen ungenügenden Widerstand entgegen zu setzen vermag, sind die Stützen beispielsweise mit Grundplatten 55 versehen.

Für die Befestigung der Fixiervorrichtung werden also im den Röhrenknochen 50 bis in den Markraum 54 reichende Öffnungen 56 (gegebenenfalls mit Gewinde 25) gebohrt, wobei der Bohrungsdurchmesser dem Durchmesser des Implantates 7, insbesondere dessen Grundplatte 55 entspricht. Das Implantat 7 weist zentral eine Stütze 52 auf, an deren distalem Ende die Grundplatte 55 befestigt ist und um die herum und die Grundplatte 55 im wesentlichen deckend ein ring- oder rohrförmiger Bereich 57 aus dem verflüssigbaren Material angeordnet ist. Das Implantat wird in die Öffnung 56 eingeführt und mit geeigneten, aussen anzwendenden Mitteln in einer vorgegebenen Tiefe festgehalten. Dann wird rund um die Stütze 52 das verflüssigbare Material 57 unter Ultraschalleinwirkung gegen die Grundplatte 55 gedrückt, so dass es zwischen Knochen 50 und Grundplatte 55 in den Markraum 54 gepresst wird und so eine formschlüssige Verbindung bildet, die die Stütze 52 in der Öffnung 56 hält. Diese Verankerung ermöglicht eine unikortikale, Verkippungs-gesicherte Befestigung der Stützen 52, was gemäss dem Stande der Technik nur mit einer bikortikalen Befestigung möglich ist.

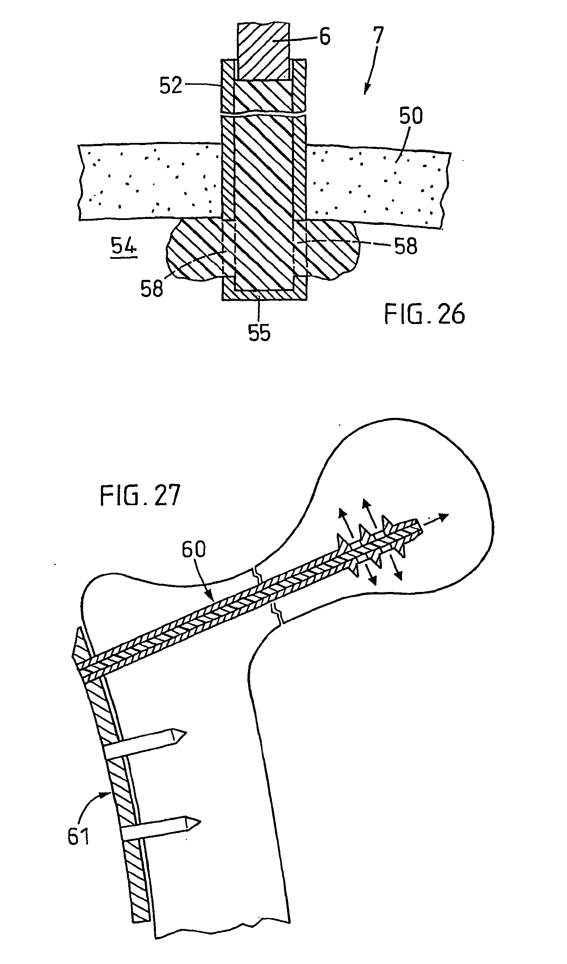

**Figur 26** zeigt eine weitere Ausführungsform des erfindungsgemässen Implantates 7, das sich insbesondere für die in der Figur 23 gezeigte Anwendung eignet. Hier ist das verflüssigbare Material, das beispielsweise ein thixotroper Zement ist, im Inneren der Stütze 52 angeordnet und über der Grundplatte 55 sind Öffnungen 58 vorgesehen, deren Grösse derart ist, dass der Zement in seiner hochviskosen Form nicht austreten kann, wohl aber in seiner durch die Wirkung des Resonators 6 verflüssigten Form. Das Ende der Stütze 52 ist also in diesem Falle als Hülse im Sinne der Figur 8 ausgebildet. Der mit Hilfe des Resonators 6 durch die Öffnungen 58 ausgepresste Zement sichert die Stütze im Markhohlraum 54 und gegebenenfalls angrenzendem Knochengewebe.

**Figur 27** zeigt eine als erfindungsgemässes Implantat ausgerüstete Zugschraube 60, die beispielsweise zusammen mit einer Trochanterplatte 61 zur Fixierung eines gebrochenen Oberschenkelhalsknochens verwendet wird. Die Zugschraube 60 ist (im Sinne einer Implantatshülse 13, Figur 8) hohl und weist mindestens in ihrem distalen Bereich Öffnungen auf, durch die ein verflüssigtes Material austreten kann, um diesen distalen Bereich beispielsweise in einem osteoporotischen Knochengewebe besser verankern zu können, als dies mit dem Gewinde der Zugschraube allein möglich ist. Das Gewinde der Schraube dient also in diesem Falle insbesondere dazu, das Gewebe im Bereiche der Bruchstelle zusammen zu ziehen, bis das distale Schraubenende durch das verflüssigbare Material im Gewebe verankert ist.

**Figur 28**. zeigt als sehr schematische Schnittdarstellung einen Röhrenknochen 50 an dem mittels erfindungsgemässem Implantat 7 ein künstliches Gelenkelement 62 befestigt ist. Der Schaft 63 des Gelenkelementes 62 und um den Schaft angeordnetes, verflüssigbares Material 57 stellen das erfindungsgemässe Implantat 7 dar, das unter Ultraschalleinwirkung in den Röhrenknochen 50 gedrückt wird, wobei das Material 57 verflüssigt wird und in Poren der Spongiosa 23 und in Unebenheiten der Innenoberfläche der Kortikalis 22 gepresst ist. Vorteilhafterweise weist der Schaft 63 im Sinne der in der Figur 17 dargestellten Platte 36 eine für eine formschlüssige Verbindung mit dem verflüssigbaren Material 57 geeignete Oberflächenstruktur auf.

Eine insbesondere vorteilhafte Ausführungsform des Schaftes 63 besteht beispielsweise aus Titan und weist eine poröse und dadurch bioaktive Oberfläche auf und ist mit einem resorbierbaren, verflüssigbaren Material umgeben. Ein derartiges Implantat hat unmittelbar nach der Implantation eine primäre Stabilität, die mindestens eine Teilbelastung erlaubt. Die primäre Stabilität wird dann abgelöst durch eine sekundäre, durch das Verwachsen von vitalem Knochengewebe mit der porösen Oberfläche des Titanschaftes 63 bedingte Stabilität. Das heisst mit anderen Worten, das künstliche Gelenkelement kann auch ohne Verwendung von Zement im wesentlichen unmittelbar nach der Implantation belastet werden, was die Regeneration des vitalen Gewebes begünstigt und einem Abbau (Osteoporose) vorbeugt. Trotzdem verwächst das vitale Gewebe in einer weiteren Phase mit dem Titanschaft.

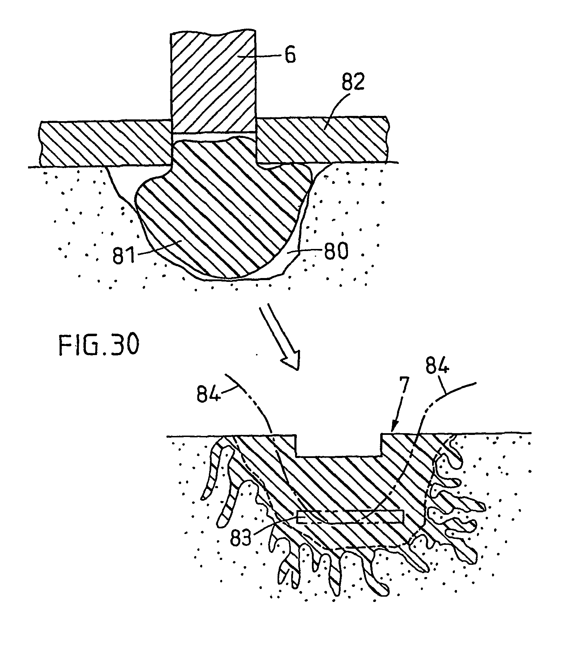

Figur 29 zeigt ebenfalls sehr schematisch ein Gelenk 70, in dessen Bereich ein Band 71 die Knochen 72 und 73 verbindet. Das Band 71 ist mit dem Knochen verwachsen, wobei diese Verbindung bei Überbelastung reissen kann. Zur Reparatur können erfindungsgemässe Implantate 7 angewendet werden, wobei beispielsweise Ausführungsformen gemäss. Figuren 2 bis 4 sinngemäss anwendbar sind. Dazu wird die Kortikalis des Gelenkknochens geöffnet und stiftförmige Implantate 7 werden durch das Band 71 getrieben und mit einem Kopf (z.B. gemäss Figur 13) aussen gesichert.

Auch Ausführungsformen mit weniger Tiefenwirkung gemäss Figuren 16 und 17 sind denkbar.

**Figur 30** zeigt abschliessend, dass ein mit einem ähnlichen aber nicht erfindungsgemässen Implantat 7 zu erstellende Verbindung nicht zwingend für die Verbindung von zwei Elementen (zwei Gewebeteile oder ein Gewebeteil mit einem künstlichen Teil) dienen muss. Es ist auch denkbar, ein Implantat für das Auffüllen einer beispielsweise durch einen Tumor verursachten Gewebeöffnung 80 zu verwenden. Dazu wird vorteilhafterweise ein Implantat 7 aus einem hochviskosen und thixotropen Material 81 verwendet, das gegebenenfalls mit Hilfe einer um die Öffnung positionierten Führung 82 die Öffnung 80 überragend in diese eingebracht wird. Der für diese Anwendung eingesetzte Resonator 6 entspricht in seinem Querschnitt dem inneren Querschnitt der Führung 82, so dass damit wie mit einem Zylinder das Material 81 in die Öffnung 80 pressbar ist. Dabei wird nicht nur die Öffnung 80 im wesentlichen lückenlos gefüllt, sondern das unter der Ultraschallwirkung flüssiger werdende Material 81 wird auch in die in die Öffnung 80 mündenden Gewebeporen gepresst und bildet darin nach dem Erstarren eine formschlüssige Verbindung, wie dies unten in der Figur 30 dargestellt ist. Diese formschlüssige Verbindung hält das Implantat 7 sicher in der Öffnung 80, ohne dass diese dafür Hinterschnitte aufzuweisen hat und ohne dass andere Haltemittel (z.B. darüber genähte Knochenhaut) eingesetzt werden müssen.

Dem verflüssigbaren Material 81 kann auch entsprechend fein aufgearbeitetes Knochenmaterial des Patienten beigemischt werden.

Wenn anstelle eines thixotropen Zementes in einem Falle, wie er in der Figur 30 dargestellt ist, ein thermoplastischer Kunststoff verwendet wird, kann die Öffnung 80 auch eine speziell hergestellte Öffnung sein und kann darin mit Hilfe des Kunststoffes ein Fixierelement 83 für einen Draht 84 oder einen Faden befestigt werden, wie das strichpunktiert in der Figur 30 (nur unten) dargestellt ist. Auch eine therapeutische Hilfsvorrichtung, wie beispielsweise ein Stimulator, kann auf diese Weise fixiert werden.

### Beispiel 1

Durch Spritzguss hergestellte Stifte aus PLLA und Polycarbonat mit einem runden Querschnitt von Durchmessern zwischen 3,5 und 4,25 mm, mit Längen von 26 bis 40 mm (ideale Länge bei 20 kHz: 35 mm) und mit stumpf zugespitzten, distalen Enden und vier, sich vom distalen Ende über 10 mm axial erstreckenden Rillen wurden mit einer Anregerfrequenz von 20 kHz in Spongiosa (Femurkopf) frisch geschlachteter Rinder verankert. Dabei wurde die über der Spongiosa liegende, dünne Kortikalis-schicht geöffnet, die Spongiosa aber nicht vorgebohrt. Die Implantate wurden mit Drücken von 60 bis 130 N gegen das Gewebe gedrückt und mit der Anregerfrequenz angeregt (Sonotrodenamplitude ca. 20 bis 25 µm). Der Vorschub wurde auf 10 mm begrenzt. Er war in jedem Falle in weniger als 2 s erreicht. Dann wurden die Implantate ohne Anregung während 5 s gehalten.

Es ergaben sich Verankerungstiefen in der Grössenordnung von 15 mm, die beim Ausreissen stärker waren als die Implantate (maximale Ausreisskräfte über 500 N). An Sensoren, die 1 mm ausserhalb von der Vorbohrung in der Kortikalis platziert wurden (1,5 mm unter der Kortikalisoberfläche), wurden ca. 10 s nach Implantationsbeginn Temperaturen von max. 44°C (ca. 22° über Raumtemperatur) gemessen. Die Übertemperaturen fielen in ca. 30 s auf den halben Wert.

Für das PLLA-Material wurde am implantierten Material gegenüber dem Material des noch nicht implantierten Implantates kein Molekulargewichtsabbau festgestellt.

## Patentansprüche

1. Implantat (7) zur Erstellung einer formschlüssigen Verbindung mit einem Gewebeteil, um diesen mit einem weiteren Gewebeteil zu verbinden oder mit einem künstlichen Element, das einen weiteren Gewebeteil ersetzt oder stützt oder eine therapeutische Hilfsvorrichtung ist, wobei das Implantat (7) mindestens teilweise aus einem durch mechanische Energie verflüssigbaren Material besteht und wobei das verflüssigbare Material am Implantat (7) derart angeordnet ist, dass es mit dem Gewebeteil in Kontakt bringbar, durch mechanische Schwingungen anregbar und gleichzeitig gegen den Gewebeteil pressbar ist, um mindestens einen Teil des verflüssigbaren Materials zu verflüssigen und in Öffnungen des Gewebeteils zu pressen.

2. Implantat nach Anspruch 1, wobei das verflüssigbare Material thermoplastisch oder thixotrop ist.

3. Implantat nach einem der Ansprüche 1 oder 2, wobei das verflüssigbare Material mindestens eine resorbierbare Komponente aufweist.

4. Implantat nach einem der Ansprüche 1 bis 3, wobei das verflüssigbare Material Zusatzstoffe für weitere Funktionen enthält.

5. Implantat nach einem der Ansprüche 1 bis 4, wobei es stift- oder dübelformig ist.

6. Implantat nach Anspruch 5, wobei es in einem proximalen Bereich mit mechanischer Energie verflüssigbares Material aufweist.

7. Implantat nach Anspruch 5 oder 6, wobei es einen kopfförmig ausgestalteten, proximalen Bereich aufweist.

8. Implantat nach Anspruch 5, wobei das verflüssigbare Material an der Implantatsoberfläche eines distalen Implantatendes und/oder an der Implantats-Mantelfläche angeordnet ist und wobei das verflüssigbare Material als Energierichtungsgeber funktionierende Stufen aufweist.

9. Implantat nach Anspruch 5, wobei das verflüssigbare Material an der Implantatsoberfläche eines distalen Implantatsendes und/oder an der Implantats-Mantelfläche angeordnet ist und wobei es einen Kern (11) aus einem nicht verflüssigbaren Material aufweist.

10. Implantat nach Anspruch 5, wobei es eine Hülse (13) aus einem nicht verflüssigbaren Material aufweist, wobei das verflüssigbare Material im Innern der Hülse angeordnet ist und wobei die Hülse für den Austritt des verflüssigten Materials Öffnungen (58) aufweist.

11. Implantat nach Anspruch 9 oder 10, wobei das verflüssigbare Material resorbierbar ist und dass der Kern (11) oder die Hülse (13) mindestens teilweise eine Oberfläche aufweist, die eine Verwachsung mit dem Gewebeteil fördert.

12. Implantat nach einem der Ansprüche 4 bis 11, wobei es an seinem proximalen Ende Haltemittel (14, 15) aufweist.

13. Implantat nach einem der Ansprüche 1 bis 4, wobei es die Form einer Platte (35 und 36) oder Folie (35) hat.

14. Implantat nach Anspruch 13, wobei die Platte oder Folie (35) einschichtig ist.

15. Implantat nach Anspruch 13, wobei die Platte (35 und 36) oder Folie eine Schicht aus dem verflüssigbaren Material und eine Schicht aus einem nicht verflüssigbaren Material aufweist und wobei das verflüssigbare Material mindestens einen Teil der einen Oberfläche des nicht verflüssigbaren Materials abdeckt und die beiden Schichten formschlüssig oder stoffschlüssig miteinander verbunden sind.

16. Implantat nach einem der Ansprüche 1 bis 15, wobei es ein Verankerungsmittel für ein künstliches Stütz- oder Fixierungselement, für ein künstliches Gelenkelement, für einen künstlichen Zahn, für eine Brücke, für eine Zahnprothese, für einen Nahtfaden, für einen Cerclage-Draht oder für eine therapeutische Hilfsvorrichtung ist.

17. Implantationsvorrichtung (1) für Implantate (7) gemäss einem der Ansprüche 1 bis 16 mit einem Generator (2), einem Schwingelement und einem Resonator (6), wobei der Generator (2) für die Anregung des Schwingelementes zu mechanischen Schwingungen ausgerüstet ist, wobei der Resonator (6) mit dem Schwingelement wirkverbunden eine Schwingeinheit (3) bildet und wobei der Resonator (6) zur Übertragung der mechanischen Schwingungen mit dem Implantat (7) wirkverbindbar und zum Pressen des Implantates (7) gegen einen Gewebeteil ausgerüstet ist, **dadurch gekennzeichnet, dass** die Vorrichtung zusätzlich eine an einem Gehäuse (5) abgestützte Implantatführung aufweist.

18. Implantätionsvorrichtung nach Anspruch 17, **dadurch gekennzeichnet, dass** die Vorrichtung für mechanische Schwingungen des Resonators (6) mit einer Frequenz von 2 bis 200 kHz ausgelegt ist.

19. Implantationsvorrichtung nach einem der Ansprüche 17 oder 18, **dadurch gekennzeichnet, dass** verschiedene Frequenzen der mechanischen Schwingungen, verschiedene Frequenzmuster und/oder verschiedene Energiepulsationen einstellbar sind.

20. Implantationsvorrichtung nach einem der Ansprüche 17 bis 19, **dadurch gekennzeichnet, dass** der Resonator (6) oder ein distaler Teil des Resonators (6) auswechselbar und sterilisierbar ist.

21. Implantationsvorrichtung nach einem der Ansprüche 17 bis 20, d**adurch gekennzeichnet, dass** der Resonator (6) zur Halterung eines stift- oder dübelförmigen Implantates (7) ausgerüstet ist.

22. Kit zur Erstellung von Verbindungen mit Gewebeteilen, welcher Kit eine Implantationsvorrichtung (1) mit einem Generator (2), einem Schwingelement und einem Resonator (6), wobei der Generator (2) für die Anregung des Schwingelementes zu mechanischen Schwingungen ausgerüstet ist, wobei der Resonator (6) mit dem Schwingelement wirkverbunden eine Schwingeinheit (3) bildet und wobei der Resonator (6) zur Übertragung der mechanischen Schwingungen mit dem Implantat (7) wirkverbindbar und zum Pressen des Implantates (7) gegen einen Gewebeteil ausgerüstet ist, und eine Mehrzahl von Implantaten (7) nach einem der Ansprüche 1 bis 13 aufweist.

23. Kit nach Anspruch 22, **dadurch gekennzeichnet, dass** das Kit eine Mehrzahl von stiftförmigen Implantaten aufweist und mindestens eine Platte (21) mit an die stiftförmigen Implantate (7) angepassten Öffnungen.

24. Kit nach Anspruch 23, **dadurch gekennzeichnet, dass** die Platte (21) aus Metall besteht, dass die stiftförmigen Implantate (7) zur Verbindung mit der Platte (21) in einem proximalen Bereich durch mechanische Energie verflüssigbares Material aufweisen und dass die Öffnungen für einen Formschluss mit dem verflüssigbaren Material ausgerüstet sind.

25. Kit nach Anspruch 24, **dadurch gekennzeichnet, dass** die Öffnungen ein Innengewinde aufweisen.

26. Kit nach Anspruch 23, **dadurch gekennzeichnet, dass** die Platte (21) und/oder ein proximales Implantatsende verflüssigbares Material aufweist, derart, dass die Platte (21) und das proximale Implantatsende durch mechanische Schwingungen miteinander verschweissbar oder verklebbar sind.

27. Kit nach Anspruch 22, **dadurch gekennzeichnet, dass** der Resonator (6) der Implantationsvorrichtung (1) an seinem distalen Ende Haltemittel aufweist und dass die Implantate (7) stift- oder dübelförmig sind und ihr proximales Ende an mit den Haltemitteln des Resonators (6) angepasste Haltemittel aufweisen.

28. Kit nach Anspruch 27, **dadurch gekennzeichnet, dass** das Haltemittel des Implantates und das Haltemittel des Resonators für eine kraftschlüssige Halterung ausgestaltet sind.

29. Kit nach einem der Ansprüche 22 bis 28, **dadurch gekennzeichnet, dass** es verschiedene, stift- oder dübelförmige Implantate aufweist sowie Resonatoren (6) oder distale Resonatorteile, deren distale Flächen proximalen Querschnitten der Implantate entsprechen.

30. Kit nach einem der Ansprüche 22 bis 29, **dadurch gekennzeichnet, dass** mindestens ein Teil der Implantate (7) stiftförmig ist und dass das Kit ferner Bohrer aufweist, deren Durchmesser an den grössten Durchmesser der Implantate (7) für eine Reibpassung angepasst ist.

31. Kit nach einem der Ansprüche 22 bis 30, **dadurch gekennzeichnet, dass** mindestens ein Teil der Implantate (7) das verflüssigbare Material in einer Hülse (13) mit Öffnungen (58) aufweisen und dass das Kit ferner mindestens einen in seinem Querschnitt an den proximalen Innenquerschnitt der Hülse (13) angepassten Resonator (6) oder distalen Resonatorteil aufweist.

32. Kit nach einem der Ansprüche 22 bis 31, **dadurch gekennzeichnet, dass** das Kit ferner Führungen (82) mit einer Öffnung und mindestes einen in seinem Querschnitt an die Öffnung angepassten Resonator (6) oder distalen Resonatorteil aufweist.

33. Kit nach einem der Ansprüche 22 bis 32, **dadurch gekennzeichnet, dass** es ferner sterile Umhüllungen für die Implantationsvorrichtung (1) aufweist.

34. Kit nach einem der Ansprüche 22 bis 33, **dadurch gekennzeichnet, dass** verschiedene Schwingfrequenzen, verschiedene Frequenzmuster und/oder verschiedene Energiepulsationen an der Implantationsvorrichtung (1) einstellbar sind und dass das Kit ferner eine Einstellungsanleitung für verschiedene Implantate aufweist.

35. Zusatzkit mit einem oder mehreren Implantaten (7) nach einem der Ansprüche 1 bis 16, welches Zusatzkit ferner eine Anleitung zur Implantation der Implantate mit Hilfe mechanischer Schwingungs-Energie aufweist.

## Claims

1. Implant (7) for creating a positive-fit connection to a tissue part for connecting the tissue part with a further tissue part or with an artificial element replacing or supporting a further tissue part or being a therapeutic auxiliary device, wherein the implant (7) at least partly consists of a material which is liquefiable by mechanical energy, wherein the liquefiable material is arranged on the implant (7) in a manner such that it can be brought into contact with the tissue part and can be excited by mechanical oscillation and simultaneously be pressed against the tissue part for liquefying at least part of the liquefiable material and for pressing it into openings of the tissue part.

2. Implant according to claim 1, wherein the liquefiable material is thermoplastic or thixotropic.

3. Implant according to one of claims 1 or 2, wherein the liquefiable material comprises at least one resorbable component.

4. Implant according to one of claims 1 to 3, wherein the liquefiable material further contains substances having further functions.

5. Implant according to one of claims 1 to 4, wherein it has the shape of a pin or dowel.

6. Implant according to claim 5, wherein it comprises material liquefiable by means of mechanical energy in a proximal region.

7. Implant according to claim 5 or 6, wherein it comprises a head-shaped proximal region.

8. Implant according to claim 5, wherein the liquefiable material is arranged on the surface of a distal implant end and/or on the circumferential implant surface and wherein the liquefiable material comprises steps acting as energy directors.

9. Implant according to claim 5, wherein the liquefiable material is arranged on the surface of a distal implant end and/or on the circumferential implant surfaces and wherein the implant comprises a core (11) of a non-liquefiable material.

10. Implant according to claim 5, wherein it comprises a sleeve (13) of a non-liquefiable material, wherein the liquefiable material is arranged in the inside of the sleeve and wherein the sleeve comprises openings (58) for the exit of the liquefied material.

11. Implant according to claim 9 or 10, wherein the liquefiable material is resorbable and wherein the core (11) or the sleeve (13) has a surface of which at least a part is equipped for furthering the growing together of bone tissue and implant.

12. Implant according to one of claims 4 to 11, wherein it comprises holding means (14, 15) arranged at a proximal implant end.

13. Implant according to one of claims 1 to 4, wherein it has the shape of a plate (35 and 36) or film (35).

14. Implant according to claim 13, wherein the plate or film (35) is of one layer.

15. Implant according to claim 15, wherein the plate (35 and 36) or film comprises a layer of the liquefiable material and a layer of a non-liquefiable material, and wherein the liquefiable material covers at least a part of the one surface of the non-liquefiable material and the two layers are connected to one another by positive fit or material fit.

16. Implant according to one of claims 1 to 15, wherein it is designed as an anchoring means for an artificial support or fixation element, for an artificial joint element, for an artificial tooth, bridge or tooth prosthesis, for a suture thread, for a cerclage wire or for a therapeutic auxiliary device.

17. Implantation device (1) for implanting implants (7) according to one of claims 1 to 16 the device comprising a generator (2), an oscillation element and a resonator (6), wherein the generator (2) is designed for exciting the oscillation element into mechanical oscillation, wherein the resonator (6) and the oscillation element are connected to form an oscillation unit (3) and wherein the resonator (6) is actively connectable to the implant (7) for transmission of the mechanical oscillations, and is designed for pressing the implant (7) against a tissue part, **characterized in that** the device further comprises an implant guide being supported on a housing (5).

18. Implantation device according to claim 17, **characterized in that** the device is designed for mechanical oscillations of the resonator (6) with a frequency of 2 to 200 kHz.

19. Implantation device according to one of claims 17 or 18, **characterized in that** the device is equipped for setting various frequencies of the mechanical oscillations, various frequency patterns and/or various energy pulsations.

20. Implantation device according to one of claims 17 to 19, **characterized in that** the resonator (6) or a distal part of the resonator (6) is exchangeable and able to be sterilized.

21. Implantation device according to one of claims 17 to 20, **characterized in that** the resonator (6) is designed for holding a pin-like or dowel-like implant (7).

22. Kit for creating connections to tissue parts, said kit comprising an implantation device (1) comprising a generator (2), an oscillation element and a resonator (6), wherein the generator (2) is designed for exciting the oscillation element into mechanical oscillation, wherein the resonator (6) and the oscillation element are connected to form an oscillation unit (3) and wherein the resonator (6) is actively connectable to the implant (7) for transmission of the mechanical oscillations, and is designed for pressing the implant (7) against a tissue part and said kit further comprising a plurality of implants (7) according to one of claims 1 to 13.

23. Kit according to claim 22, **characterized in that** the kit comprises a plurality of pin-shaped implants and at least one plate (21) with openings adapted to the pin-shaped implants (7).

24. Kit according to claim 23, **characterized in that** the plate (21) consists of metal, that for connection to the plate (21) the pin-shaped implants (7) comprise material liquefiable by means of mechanic energy in a proximal region and that the openings are equipped for positive engagement with the liquefiable material.

25. Kit according to claim 24, **characterized in that** the openings comprise an internal thread.

26. Kit according to 23, **characterized in that** the plate (21) and/or a proximal implant-end comprise liquefiable material, such that the plate (21) and the proximal implant-end are connectable by means of welding or gluing.

27. Kit according to claim 22, **characterized in that** the resonator (6) of the implantation device (1) comprises holding means at its distal end and that the implants (7) are pin-like or dowel-like and their proximal end comprises holding means adapted to the holding means of the resonator (6).

28. Kit according to claim 27, **characterized** the holding means of the implant and the holding means of the resonator are designed for a friction-fit engagement.

29. Kit according to one of the claims 22 or 29, **characterized in that** it comprises various pin-like or dowel-like implants as well as resonators (6) or distal resonator parts, whose distal surfaces correspond to the proximal cross sections of the implants.

30. Kit according to one of claims 22 to 29, **characterized in that** at least a part of the implants (7) is pin-like and that the kit further comprises drills whose diameter is adapted to the largest diameter of the implants (7) for a press fit implantation.

31. Kit according to one of claims 22 to 30, **characterized in that** at least a part of the implants (7) comprises the liquefiable material in a sleeve (13) having openings (58) and that the kit further comprises at least one resonator (6) or distal resonator part, which in its cross section is adapted to the proximal inner cross section of the sleeve (13).

32. Kit according to one of claims 22 to 31, **characterized in that** the kit further comprises guides (82) with an opening, and at least one resonator (6) or distal resonator part, which in its cross section is adapted to the opening.

33. Kit according to one of claims 22 to 32, **characterized in that** it further comprises sterile coverings for the implantation device (1).

34. Kit according to one of claims 22 to 33, **characterized in that** the implantation device (1) is equipped for setting various oscillation frequencies, various frequency patterns and/or various energy pulsations and that the kit further comprises setting instructions for various implants.

35. Addition kit with one or more implants (7) according to one of claims 1 to 16, said addition kit further comprising instructions for implanting the implants with the help of mechanical oscillation energy.

## Revendications

1. Implant (7) destiné à établir une liaison en correspondance géométrique avec une partie d'un tissu pour relier cette dernière à une autre partie de tissu ou à un élément synthétique qui remplace ou soutient une autre partie de tissu ou est un dispositif thérapeutique auxiliaire, l'implant (7) étant constitué au moins en partie d'un matériau qui peut être liquéfié par l'action d'une énergie mécanique, le matériau liquéfiable étant disposé sur l'implant (7) de telle sorte qu'il puisse être mis en contact avec la partie de tissu, être excité par des oscillations mécaniques et en même temps être repoussé contre la partie de tissu pour liquéfier au moins une partie du matériau liquéfiable et la faire pénétrer dans les ouvertures de la partie de tissu.

2. Implant selon la revendication 1, dans lequel le matériau liquéfiable est thermoplastique ou thixotrope.

3. Implant selon l'une des revendications 1 ou 2, dans lequel le matériau liquéfiable présente au moins un composant résorbable.

4. Implant selon l'une des revendications 1 à 3, dans lequel le matériau liquéfiable contient des additifs destinés à assurer d'autres fonctions.

5. Implant selon l'une des revendications 1 à 4, configuré en forme de tige ou de cheville.

6. Implant selon la revendication 5, qui présente dans une partie proximale un matériau apte à être liquéfié sous l'action d'une énergie mécanique.

7. Implant selon les revendications 5 ou 6, qui présente une partie proximale configurée en forme de tête.

8. Implant selon la revendication 5, dans lequel le matériau liquéfiable est disposé à la surface de l'extrémité distale de l'implant et/ou sur la surface d'enveloppe de l'implant, le matériau liquéfiable présentant des épaulements qui jouent le rôle de guides d'orientation de l'énergie.

9. Implant selon la revendication 5, dans lequel le matériau liquéfiable est disposé à la surface de l'extrémité distale de l'implant et/ou sur la surface d'enveloppe de l'implant, l'implant présentant une âme (11) en un matériau non liquéfiable.

10. Implant selon la revendication 5, qui présente une douille (13) en un matériau non liquéfiable, le matériau liquéfiable étant disposé à l'intérieur de la douille et la douille présentant des ouvertures (58) qui permettent la sortie du matériau liquéfié.

11. Implant selon les revendications 9 ou 10, dans lequel le matériau liquéfiable est résorbable, l'âme (11) ou la douille (13) présentant au moins en partie une surface qui favorise la croissance avec la partie du tissu.

12. Implant selon l'une des revendications 4 à 11, qui présente des moyens de maintien (14, 15) à son extrémité proximale.

13. Implant selon l'une des revendications 1 à 4, qui a la forme d'une plaque (35 et 36) ou d'une feuille (35).

14. Implant selon la revendication 13, dans lequel la plaque ou la feuille (35) sont en une seule couche.

15. Implant selon la revendication 13, dans lequel la plaque (35 et 36) ou la feuille présentent une couche constituée du matériau liquéfiable et une couche d'un matériau non liquéfiable, le matériau liquéfiable recouvrant au moins une partie d'une surface du matériau non liquéfiable et les deux couches étant reliées l'une à l'autre en correspondance géométrique ou en correspondance de matière.

16. Implant selon l'une des revendications 1 à 15, qui est un moyen d'ancrage pour un élément artificiel de soutien ou de fixation, pour un élément artificiel d'articulation, pour une dent artificielle, pour un pontage, pour une prothèse de dent, pour un fil de suture, pour un fil de cerclage ou pour un dispositif thérapeutique auxiliaire.

17. Dispositif (1) d'implantation d'implants (7) selon l'une des revendications 1 à 16, qui présente un générateur (2), un élément oscillant et un résonateur (6), le générateur (2) étant équipé pour induire des oscillations mécaniques dans l'élément oscillant, le résonateur (6) formant en coopération avec l'élément oscillant une unité oscillante (3), le résonateur (6) pouvant coopérer avec l'implant (7) pour lui transmettre des oscillations mécaniques et pour enfoncer l'implant (7) contre une partie de tissu, **caractérisé en ce que** le dispositif présente de plus un guide d'implant qui s'appuie sur un boîtier (5).

18. Dispositif d'implantation selon la revendication 17,
**caractérisé en ce que** le dispositif est conçu pour faire osciller mécaniquement le résonateur (6) à une fréquence de 2 à 200 kHz.

19. Dispositif d'implantation selon l'une des revendications 17 ou 18, **caractérisé en ce que** l'on peut y régler différentes fréquences des oscillations mécaniques, différents motifs de fréquences et/ou différentes pulsations d'énergie.

20. Dispositif d'implantation selon l'une des revendications 17 à 19, **caractérisé en ce que** le résonateur (6) ou une partie distale du résonateur (6) peuvent être remplacés et stérilisés.

21. Dispositif d'implantation selon l'une des revendications 17 à 20, **caractérisé en ce que** le résonateur (6) est équipé pour retenir un implant (7) en forme de tige ou de cheville.

22. Trousse de réalisation de liaisons à des parties de tissu, laquelle trousse est dotée d'un dispositif d'implantation (1) doté d'un générateur (2), d'un élément oscillant et d'un résonateur (6), le générateur (2) étant prévu pour créer des oscillations mécaniques dans l'élément oscillant, le résonateur (6) formant une unité oscillante (3) en coopération avec l'élément oscillant et le résonateur (6) pouvant coopérer avec l'implant (7) pour y induire des oscillations mécaniques et étant équipé pour repousser l'implant (7) contre une partie de tissu, et de plusieurs implants (7) selon l'une des revendications 1 à 13.

23. Trousse selon la revendication 22, **caractérisée en ce que** la trousse présente plusieurs implants en forme de tige et au moins une plaque (21) dotée d'ouvertures adaptées aux implants (7) en forme de tige.

24. Trousse selon la revendication 23, **caractérisée en ce que** la plaque (21) est réalisée en métal, **en ce que** pour être reliés à la plaque (21), les implants (7) présentent dans une partie proximale un matériau qui peut être liquéfié par l'action d'une énergie mécanique et **en ce que** les ouvertures sont prévues de manière à être en correspondance géométrique avec le matériau liquéfiable.

25. Trousse selon la revendication 24, **caractérisée en ce que** les ouvertures présentent un filet intérieur.

26. Trousse selon la revendication 23, **caractérisée en ce que** la plaque (21) et/ou l'extrémité proximale de l'implant présentent un matériau liquéfiable, de telle sorte que la plaque (21) et l'extrémité proximale de l'implant puissent être soudées l'une à l'autre ou collées l'une à l'autre par des oscillations mécaniques.

27. Trousse selon la revendication 22, **caractérisée en ce que** le résonateur (6) du dispositif d'implantation (1) présente à son extrémité distale des moyens de maintien et **en ce que** les implants (7) sont en forme de tige ou de cheville et présentent à leur extrémité proximale des moyens de maintien adaptés aux moyens de maintien du résonateur (6).

28. Trousse selon la revendication 27, **caractérisée en ce que** le moyen de maintien de l'implant et le moyen de maintien du résonateur sont configurés pour assurer un maintien en correspondance mécanique.

29. Trousse selon l'une des revendications 22 à 28, **caractérisée en ce qu'**elle présente différents implants en forme de tige ou de cheville ainsi que des résonateurs (6) ou des parties distales de résonateur dont la surface distale correspond à la section transversale proximale des implants.

30. Trousse selon l'une des revendications 22 à 29, **caractérisée en ce qu'**au moins une partie des implants (7) est en forme de tige et **en ce que** la trousse présente en outre des forets dont le diamètre est adapté au plus grand diamètre des implants (7) en vue d'une adaptation par frottement.

31. Trousse selon l'une des revendications 22 à 30, **caractérisée en ce qu'**au moins une partie des implants (7) présente le matériau liquéfiable dans une douille (13) dotée d'ouvertures (58) et **en ce que** la trousse présente en outre au moins un résonateur (6) ou une partie distale de résonateur dont la section transversale est adaptée à la section transversale intérieure proximale de la douille (13).

32. Trousse selon l'une des revendications 22 à 31, **caractérisée en ce que** la trousse présente en outre des guides (82) dotés d'une ouverture et au moins un résonateur (6) ou une partie distale de résonateur dont la section transversale est adaptée à l'ouverture.

33. Trousse selon l'une des revendications 22 à 32, **caractérisée en ce qu'**elle présente en outre des emballages stériles pour le dispositif d'implantation (1).

34. Trousse selon l'une des revendications 22 à 33, **caractérisée en ce que** différentes fréquences d'oscillation, différents motifs de fréquence et/ou différentes pulsations d'énergie peuvent être réglés sur le dispositif d'implantation (1) et **en ce que** la trousse possède en outre des indications de réglage pour différents implants.

35. Trousse complémentaire qui présente un ou plusieurs implants (7) selon l'une des revendications 1 à 16, la trousse complémentaire possédant en outre des indications pour l'implantation des implants à l'aide d'une énergie mécanique apportée par oscillations.
